(19) Europäisches Patentamt
European Patent Office
Office européen des brevets

(11) Publication number: 0 299 973 B1

(12) # EUROPEAN PATENT SPECIFICATION

(45) Date of publication of patent specification: **17.06.92**

(51) Int. Cl.$^5$: **A61K 31/655**, C07D 209/14, C07D 307/81, C07D 311/12, C07D 333/58, C07D 401/12

(21) Application number: **87902989.0**

(22) Date of filing: **03.04.87**

(86) International application number: **PCT/US87/00698**

(87) International publication number: **WO 87/06133 (22.10.87 87/23)**

(54) **ANTHELMINTIC ACYLHYDRAZONES, METHOD OF USE AND COMPOSITIONS.**

(30) Priority: **07.04.86 US 849034**

(43) Date of publication of application: **25.01.89 Bulletin 89/04**

(45) Publication of the grant of the patent: **17.06.92 Bulletin 92/25**

(84) Designated Contracting States: **AT BE CH DE FR GB IT LI LU NL SE**

(56) References cited:
**WO-A-86/05982**
**US-A- 2 775 593**
**US-A- 3 882 149**

**Chemical Abstracts, vol. 92, no. 15, 14 April 1980, (Columbus, Ohio, US), A. Tajana et al.: "New alkylamino, dialkylamino and arylalkylaminoacethydrazones with antiinflammatory activity", page 22, abstract 121571v, Boll. Chim. Farm. 1979, 118(7), 397-406**

(73) Proprietor: **THE UPJOHN COMPANY**
**301 Henrietta Street**
**Kalamazoo, Michigan 49001(US)**

(72) Inventor: **RECTOR, Douglas, L.**
**6075 Litchfield Lane**
**Kalamazoo, MI 49009(US)**
Inventor: **CONDER, George, A.**
**6835 East "F" Avenue**
**Richland, MI 49083(US)**
Inventor: **FOLZ, Sylvester D.**
**6209 Enola Drive**
**Kalamazoo, MI 49004(US)**

(74) Representative: **Perry, Robert Edward et al**
**GILL JENNINGS & EVERY 53-64 Chancery Lane**
**London WC2A 1HN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Chemical Abstracts, vol. 67, 1967, (Columbus, Ohio, US), p 3080, abstract 32590s & ES A 324609

Chemical Abstracts, vol. 96, no. 17, 26 April 1982, (Columbus, Ohio, US), S. Misra et al.: "Synthesis of 2-substituted phenyl-3-aryloxyacetyl hydrazono methylenyl-indoles as central nervous system active agents, p 735, abstract 142622k, J. Chem. Soc. Pak. 1981, 3/4, 199-202

## Description

### SUMMARY OF THE INVENTION

This invention pertains to compounds as defined in the claims for use in killing and controlling worms (Helminths), and new formulations for killing and controlling worms in animals, and new chemical compounds as defined in the claims. The invention is more particularly directed to the use of certain acylhydrazones, to new anthelmintic formulations comprising the same, and to new acylhydrazones as defined below.

### BACKGROUND OF THE INVENTION

The diseases or groups of diseases described generally as helminthiasis are due to infection of the animal with parasitic worms known as helminths. Helminthiasis and helminthosis are prevalent and may lead to serious economic problems in valuable warm-blooded domestic animals such as sheep, swine, cattle, goats, dogs, cats, horses, poultry and man. Among the helminths, the groups of worms known as nematodes, trematodes and cestodes cause widespread and often-times serious infections in various species of animals including man The most common genera of nematodes, trematodes and cestodes infecting the animals referred to above are Dictyocaulus, Haemonchus, Trichostrongylus, Ostertagia, Nematodirus, Cooperia, Bunostomum, Oesophagostomum, Chabertia, Strongyloides, Trichuris, Fasciola, Dicrocoelium, Enterobius, Ascaris, Toxascaris, Toxocara, Ascaridia, Capillaria, Heterakis, Ancylostoma, Uncinaria, Dirofilaria, Onchocerca, Taenia, Moniezia, Dipylidium, Metastrongylus, Triodontophorus, Macracanthorhynchus, Hyostrongylus, and Strongylus. Some of these genera attack primarily the intestinal tract while others, inhabit the stomach, lungs, liver and subcutaneous tissues. The parasitic infections causing helminthiasis and helminthosis lead to anemia, malnutrition, weakness, weight loss, unthriftiness, severe damage to the gastrointestinal tract wall and, if left to run their course, may result in death of the infected animals.

WO-A-8605982 is in the state of the art according to Article 54(3) EPC; it discloses quinolinyl acylhydrazones and their use for killing internal parasites, especially nematodes and cestodes, affecting warm-blooded animals.

Among the acylhydrazones of formula I:
acetic acid [(1H-indol-3-yl)methylene]hydrazide (CA RN 1016-49-5);
4-aminobenzoic acid [(1H-indol-3-yl)methylene]hydrazide (CA RN 10245-42-8);
4-methoxybenzoic acid [(1-methylindol-2-yl)methylene]hydrazide (CA RN 15565-86-3);
4-morpholineacetic acid (2-benzofuranylmethylene)hydrazide (CA RN 81111-25-3);
(2-chlorophenoxy)acetic acid (1H-indol-3-ylmethylene)hydrazide (CA RN 81111-25-3);
(4-chlorophenoxy)acetic acid (1H-indol-3-ylmethylene)hydrazide (CA RN 81111-26-4);
(2-chlorophenoxy)acetic acid [(2-methoxy-1H-indol-3-yl)methylene]hydrazide (CA RN 81122-74-9);
(4-chlorophenoxy)acetic acid [(2-methoxy-1H-indol-3-yl)methylene]hydrazide (CA RN 81122-75-0);
methyl (1H-indol-3-ylmethylene)carbazate (CA RN 88692-99-3);
phenylmethyl [1-(1H-indol-3-yl)propylidene]carbazate (CA RN 32947-16-3);
1-piperidineacetic acid [(2-methyl-3-benzofuranyl)methylene]hydrazide (CA AN 35806-44-1);
1-pyrrolidineacetic acid (2-benzofuranylmethylene)hydrazide (CA RN 35806-70-3);
1-piperidineacetic acid (2-benzofuranylmethylene)hydrazide (CA RN 35806-85-0);
1-pyrrolidineacetic acid [(2-methyl-3-benzofuranyl)methylene]hydrazide (CA RN 35802-00-9);
formic acid [(1H-indol-2-yl)methylene]hydrazide (CA RN 50335-88-1);
ethyl (1H-indol-2-ylmethylene)carbazate (CA RN 50335-89-2);
ethyl [1-(2-benzofuranyl)ethylidene]carbazate (CA RN 56968-94-6);
ethyl [1-(5-chloro-2-benzofuranyl)ethylidene]carbazate (CA RN 56968-95-7);
1,1-dimethylethyl (1H-indol-3-ylmethylene)carbazate (CA RN 57699-52-2);
4-aminobenzoic acid [(1H-indol-3-yl)methylene]hydrazide (CA RN 15641-21-1);
butyric acid [(1H-indol-3-yl)methylene]hydrazide (CA RN 15641-24-4);
propionic acid [(1H-indol-3-yl)methylene]hydrazide (CA RN 15641-25-5);
ethyl [(1H-indol-3-yl)methylene]carbazate (CA RN 15641-27-7);
formic acid [(1-methylindol-2-yl)methylene]hydrazide (CA RN 15641-28-8);
acetic acid [(1-methylindol-2-yl)methylene]hydrazide (CA RN 15641-29-9);
propanoic acid [(1-methylindol-2-yl)methylene]hydrazide (CA RN 15641-30-2);
butanoic acid [(1-methylindol-2-yl)methylene]hydrazide (CA RN 15641-31-3);

2-methylpropanoic acid [(1-methylindol-2-yl)methylene]hydrazide (CA RN 15641-32-4);
phenylacetic acid [(1-methylindol-2-yl)methylene]hydrazide (CA RN 15641-34-6);
4-chlorobenzoic acid [(1-methylindol-2-yl)methylene]hydrazide (CA RN 15641-36-8);
1-naphthaleneacetic acid [(1-methylindol-2-yl)methylene]. hydrazide (CA RN 15641-38-0);
ethyl [(1-methylindol-2-yl)methylene]carbazate (CA RN 15565-88-5);
4-chlorobenzoic acid [(1H-indol-3-yl)methylene]hydrazide (CA RN 15641-01-7);
2-chlorobenzoic acid [(1H-indol-3-yl)methylene]hydrazide (CA RN 15641-03-9);
3-chlorobenzoic acid [(1H-indol-3-yl)methylene]hydrazide (CA RN 15641-04-0);
2,4-dichlorobenzoic acid [(1H-indol-3-yl)methylene]hydrazide (CA RN 15641-05-1);
4-bromobenzoic acid [(1H-indol-3-yl)methylene]hydrazide (CA RN 15641-06-2);
2-methylpropanoic acid [(1H-indol-3-yl)methylene]hydrazide (CA RN 15641-12-0);
4-methoxybenzoic acid [(1H-indol-3-yl)methylene]hydrazide (CA RN 15641-15-3);
phenylacetic acid [(1H-indol-3-yl)methylene]hydrazide (CA RN 15641-17-5);
1-naphthaleneacetic acid [(1H-indol-3-yl)methylene]hydrazide (CA RN 15641-18-6);
2-furoic acid [(1H-indol-3-yl)methylene]hydrazide (CA RN 15641-19-7);
2-naphthaleneacetic acid [(1H-indol-3-yl)methylene]hydrazide (CA RN 15718-86-2):
ethyl [1-(1H-indol-3-yl)ethylidene]carbazate (CA RN 32927-03-0);
phenylmethyl 3-[(1H-indol-3-yl)methylene]carbazate (CA RN 32927-05-2);
phenylmethyl [1-(1H-indol-3-yl)ethylidene]carbazate (CA RN 32927-06-3);
ethyl [(1-methylindol-3-yl)methylene]carbazate (CA RN 32927-89-2);
ethyl [1-(1H-indol-3-yl)propylidene]carbazate (CA RN 32947-13-0) are known. See S. Swaminathan and K. Narasinhan, Indiana J. Chem., 2(10), 423-424 (1964); Chem. Abstr. 62:3995F; F. Fujikawa, et al, 86, 861-864 (1966); Chem Abstr. 65:20087b; I.P. Singh et al, Arch. Pharm. 317(17), 609-614 (1985); A. Alemany et al, Bull. Soc. Chim. Fr., (3), 780-786 (1967); E. Fernandez Alvarez, et al, Span. ES 324609, 16 Dec 1966, 24 Mar 1966; Chem. Abstr. 67(7):32590s; A. Tajana et al, Boll. Chim. Farm., 118(7), 397-406 (1979); Chem. Abstr. 92(15):121571v; S. Misra et al, J. Chem. Soc. Pak., 3(4), 199-202 (1981); Chem. Abstr. 96(17)-:142622h; P. Benko et al, Ger. Offen. DE3316316A1, 10 Nov 1983; Chem. Abstr. 100(9):68022x; M. Bernabe et al, Bull. Soc. Chim. Fr., (5), 1882-1887 (1971); Chem. Abstr. 75(9):63537z; A. Tajana and R. Pozzi, J. Med. Chem., 14, 1017-1020 (1971);M. Robba et al, Bull. Soc. Chim. Fr., (3-4, Pt 2), 333-336 (1977); Chem. Abstr. 88(3):22764w; M. Robba et al, Tetrehedron Lett., (35), 3235-3238 (1973); M. Robba et al, J. Heterocycl. Chem., 14(8), 1365-1368 (1977); J.P. Dusza et al, U.S. 3882149, 6 May 1975; Chem. Abstr. 83-(13): 114190f; A.S. Dutta and J.S. Morley, J. Chem. Soc. Perkins Trans., 1(17), 1712-1720 (1975); N.R. El-Rayyes amd A.H. Katrib, J. Chem. Eng. Data, 28(1), 132-134 (1983); N.R. El-Rayyes and F.M. Al-Kharafi, Egypt, J. Chem., Vol. date 1980, 23(2), 151-156 (1981); Chem. Abstr. 96(17):142575x; R. Jurado et al, Arch. Inst. Farmacol. Exp., 18(2), 49-58 (1966); Chem. Abstr. 69(19):75350x.

## Summary of the Invention

Acylhydrazones, including hydrates or pharmaceutically-acceptable salts thereof, of formula I as defined in claim 1, are for use in killing parasitic worms in humans and valuable warm-blooded domestic animals, according to a first aspect of this invention.

According to a second aspect of this invention, novel acylhydrazones, including hydrates or pharmaceutically-acceptable salts thereof, are as defined in claim 1, excluding the known acylhydrazones of formula I recited above.

## Detailed Description of the Invention

The carbon content of various hydrocarbon-containing moieties is indicated by a prefix designating the minimum and maximum number of carbon atoms in the moiety. Thus ($C_1$-$C_3$) alkyl refers to alkyl of one to 3 carbon atoms, i.e. methyl, ethyl, propyl, and isopropyl.

Halogen atom (halo) refers to a bromo, chloro, iodo or fluoro atom.

Heteroaromatic refers to pyrrole, 3-pyridine (pyridin-3-yl), thiophene or furan, as well as the N-oxides, hydrates and pharmaceutically acceptable salts thereof. When Y is sulfur, the term heteroaromatic also includes 2-pyridine and 4-pyridine, preferably 4-pyridine, as well as the N-oxides, hydrates and pharmaceutically acceptable salts thereof.

Pharmaceutically-acceptable refers to those properties and/or substances which are acceptable to the patient from a pharmacologically-toxicological point of view and to the manufacturing pharmaceutical chemist from a physical-chemical point of view regarding composition, formulation, stability, patient

acceptance and bioavailability.

Examples of $C_1$-$C_4$ alkyl are methyl, ethyl, propyl, butyl and isomeric forms thereof. Examples of $C_1$-$C_3$ alkoxy are methoxy, ethoxy, propoxy and isomeric forms thereof. Examples of phenoxy substituted with one, 2 or 3 $C_1$-$C_4$ alkyl are (o-, m-, or p-)tolyl, (o-, m-, or p-)ethylphenyl, p-tert-butylphenyl, 2,5-dimethylphenyl, 2,6-dimethylphenyl, 2,4-dimethylphenyl, (2,3,4-, 2,3,5-, 2,3,6-, 2,4,6- or 2,4,5-)trimethylphenyl.

Examples of $C_2$-$C_6$ dialkylamino are dimethylamino, diethylamino, methylethylamino, dipropylamino and ethylpropylamino.

Examples of phenyl($C_1$-$C_3$)alkyl are benzyl, phenylethyl and phenylpropyl. Examples of phenyl($C_1$-$C_3$)-alkyl substituted with halo or trifluoromethyl include 4-chlorobenzyl, 2-chlorophenylethyl or p-trifluoromethyl.

Examples of naphthyl($C_1$-$C_3$)alkyl include 2-naphthylmethyl and 1-naphthylethyl. Examples of substituted naphthyl($C_1$-$C_3$)alkyl is (3,8-dichloro-1-naphthyl)methyl; (4-chloro-1-naphthyl)methyl; and (4-methoxy-1-naphthyl)methyl. Examples of substituted naphthyl include 3,6-dichloro-1-naphthyl; 3,5-dichloro-2-naphthyl; 6-methyl-2-naphthyl; and 4,6-dichloro-1-naphthyl.

Examples of substituted cyclo($C_3$-$C_{10}$)alkyl are chrysanthemyl, 1-methylcyclopropyl and 2-methylcyclopropyl. Examples of cyclo($C_3$-$C_{10}$)alkyl($C_1$-$C_4$)alkyl are 2-cyclohexylethyl and cyclohexylmethyl. An example of substituted cyclo($C_3$-$C_6$)alkyloxy is menthyl.

Examples of bridged polycyclic hydrocarbon substituents of six to 10 nuclear carbons, optionally substituted with one, 2 or 3 ($C_1$-$C_3$) alkyl groups include exo or endo-2-norbonyl, bicyclo[2,2,2]oct-1-yl, and 1-adamantyl.

Examples of perhalo ($C_1$-$C_7$) alkyl include trifluoromethyl, n-heptafluoropropyl and n-undecafluoropentyl.

Preferred acylhydrazones of Formula I are indolyl acylhydrazones (IC). Preferred $R_1$ and $R_2$ include hydrogen, methyl or a bromo atom.

$R_3$ is preferably hydrogen.

Preferred X include $C_1$-$C_4$ alkyl; cyclo($C_3$-$C_6$)alkyl; $C_1$-$C_4$ alkoxy; and 3-pyridinyl. Most preferably X includes cyclobutyl; t-butoxy; ethoxy; ethyl; and 3-pyridinyl.

Z is preferably hydrogen.

The acylhydrazones of this invention (formula I) are readily prepared by reacting the appropriate ketone/aldehyde (II) with the appropriate acylhydrazide/carbazate (III) (Chart A, Scheme A), or by heating the heteroaromatic ketone/aldehyde (II) with the appropriate hydrazine (IV) to form the hydrazone intermediate (V) which is then acylated with the halide or anhydride (VI) to form the acylhydrazone (I) (Chart A, Scheme B).

The reaction of Scheme A is carried out in the presence of a suitable solvent such as, for example, water, alcohols, ethers, halogenated hydrocarbons or hydrocarbons; specific examples include methanol, ethanol, isopropanol, propanol, hexane, tetrahydrofuran, dioxane and methylene chloride, preferably ethanol. A catalyst such as glacial acetic acid, hydrochloric acid, sulfuric acid or p-toluenesulfonic acid can be utilized to enhance the yield/rate of the reaction, particularly when $R_3$ is alkyl of 3 or more atoms, arylalkyl or aryl.

The acylation reaction of Scheme B is carried out in the presence of a suitable base such as an organic tertiary amine or an inorganic base, for example, triethylamine or sodium carbonate, or preferably, pyridine. The base may also be the solvent.

The starting compounds are known or can be readily prepared by known methods. R. L. Frank and C. Weatherbee, J. Am. Chem. Soc., 70, 3482-3 (1948); N. B. Mahishi, et al., J. Indian Chem. Soc., 42, 67-74 (1965) and M. Ogata and H. Kano, Chem. Pharm. Bull (Tokyo), 11, 32 (1963).

The following detailed examples/procedures describe how to prepare various acylhydrazones of the invention and are to be construed merely illustrative.

Procedure 1 Preparation of β-morpholinopropionyl [(1H-indol-3-yl)methylene]hydrazide hydrate. Compound 1.

A mixture of 3.46 gm (0.02 mole) of β-morpholinopropionylhdyrazide, 2.90 gm (0.02 mole) of indole-3-carboxaldehyde and 100 ml of ethanol is refluxed 6 hr. The solvent is evaporated. The residue is dissolved in hot dimethylformamide and filtered. The filtrate is diluted with ethyl acetate to the cloud point, cooled, then chilled. The product is collected, washed with ether and dried to yield 4.63 gm (74%) of the title compound having a melting point of 235.2° C.

| | | | |
|---|---|---|---|
| Analysis Calcd: Found: | C, 61.42; C, 61.41; | H, 6.81; H, 6.71; | N, 17.91 N, 17.47 |

Procedure 2 Preparation of 3-chlorobenzoic acid [(1H-indol-3-yl)methylene]hydrazide. Compound 2.

A mixture of 7.26 gm (0.05 mole) of indole-3-carboxaldehyde, 8.52 gm (0.05 mole) 3-chlorobenzoic acid hydrazide and 50 ml of ethanol is refluxed 12 hr. The reaction mixture is cooled. The product is collected, washed with water and dried to yield 11.55 gm (77%) of the title compound having a melting point of 224.3 ° C.

| | | | | |
|---|---|---|---|---|
| Analysis Calcd: Found: | C, 64.54; C, 64,72; | H, 4.06; H, 4.07; | N, 14.11; N, 14.14; | Cl, 11.91 Cl, 11.77 |

Procedure 3 Preparation of 1,1-dimethylethyl [(1H-indol-3-yl)methylene]carbazate. Compound 4.

A mixture of 7.26 gm (0.05 mole) of indole-3-carboxaldehyde, 6.81 gm (0.05 mole) of tert-butylcarbazate and 50 ml of ethanol is refluxed 10 hr. The solvent is evaporated to give an oil which solidifies on standing. The product is crystallized from aqueous ethanol to yield 8.65 gm (67%) of the title compound having a melting point of 136.2 ° C.

| | | | |
|---|---|---|---|
| Analysis Calcd: Found: | C, 64.84; C, 64.47; | H, 6.61; H, 6.52; | N, 16.21 N, 15.95 |

Procedure 4 Preparation of ethyl[1-(2-benzofuranyl)ethylidene]carbazate. Compound 8.

A mixture of 6.25 gm (0.06 mole) of ethyl carbazate, 9.61 gm (0.06 mole) of 2-acetylbenzofuran and 100 ml of aqueous ethanol is refluxed 6 hr. The reaction mixture is cooled. The crude product is collected and recrystallized from alcohol to yield 13.34 gm (90%) of the title compound having a melting point of 167.8 ° C.

| | | | |
|---|---|---|---|
| Analysis Calcd: Found: | C, 63.41; C, 63.56; | H, 5.69; H, 5.71; | N, 11.38 N, 11.41 |

Procedure 5 Preparation of 2-chlorobenzoic acid [(1H-indol-3-yl)methylene]hydrazide. Compound 15.

A mixture of 4.36 gm (0.03 mole) of indole-3-carboxaldehyde, 5.10 gm (0.03 mole) of 2-chlorobenzhydrazide, 100 ml of ethanol and 5 ml of glacial acetic acid is refluxed 3 hr. The mixture is diluted with sufficient dimethylformamide to give a solution. The hot solution is filtered. The filtrate is diluted with water to the cloud point and cooled, then chilled. The product is collected, washed with water and dried to yield 8.25 gm (93%) of the title compound having a melting point of 231.4 ° C.

| | | | |
|---|---|---|---|
| Analysis Calcd: Found: | C, 64.65; C, 64.81; | H, 4.04; H, 3.83; | N, 14.14 N, 14.21 |

Procedure 6 Preparation of butanoic acid [1-(1-benzylindol-3-yl)propylidene]hydrazide. Compound 49.

A mixture of 11.85 gm (0.045 mole) of 1-benzylindol-3-yl ethyl ketone, 4.6 gm (0.045 mole) of butanoic acid hydrazide, 250 ml of ethanol and 12 drops of concentrated hydrochloric acid is refluxed 6 hr. The hot

solution is filtered. The filtrate is cooled and chilled. This product is collected and dried to yield 2.18 gm (14%) of the title compound having a melting point of 277.4° C.

| | | | |
|---|---|---|---|
| Analysis Calcd: | C, 7605; | H, 7.25; | N, 12.09 |
| Found: | C, 76.00; | H, 7.42; | N, 12.07 |

Procedure 7 Preparation of isonicotinic acid [1-(1-benzylindol-3-yl)propylidene]hydrazide. Compound 58.

A mixture of 5.49 gm (0.04 mole) of isonicotinic acid hydrazide, 10.53 gm (0.04 mole) of 1-benzylindol-3-yl ethyl ketone, 200 ml of ethanol and 12 drops of glacial acetic acid is refluxed 20 hr. The reaction mixture is cooled. The product is collected and dried to yield 6.31 gm (41%) of the title compound having a melting point of 200.8° C.

| | | | |
|---|---|---|---|
| Analysis Calcd: | C, 75.37; | H, 5.80; | N, 14.65 |
| Found: | C, 75.79; | H, 5.85; | N, 14.08 |

Procedure 8 Preparation of methyl[(2-methylindol-3-yl)methylene]cabazate. Compound 161.

A mixture of 7.16 gm (0.045 mole) of 2-methylindol-3-carboxaldehyde, 4.04 gm (0.045 mole) of methyl carbazate and 100 ml of absolute ethanol is refluxed 8 hr. The hot solution is diluted with water to the cloud point, then cooled. The product is collected and dried to yield 7.79 gm (71%) of the title compound having a melting point of 152.7° C.

| | | | |
|---|---|---|---|
| Analysis Calcd: | C, 59.10; | H, 5.95; | N, 17.23 |
| Found: | C, 58.66; | H, 5.56; | N, 17.03 |

Procedure 9 Ethyl [1-(2-oxo-2H-1-benzopyran-3-yl)ethylidene]carbazate. Compound 248.

A mixture of 5.73 gm (0.055 mole) of ethyl carbazate, 10.34 gm (0.055 mole) of 3-acetylcoumarin and 130 ml of aqueous ethanol is refluxed 8 hr. The mixture is chilled. The white crystals are collected and dried to give 6.98 gm (46%) of the title compound having a melting point of 170.2° C.

| | | | |
|---|---|---|---|
| Analysis Calcd: | C, 61.31; | H, 5.11; | N, 10.22 |
| Found: | C, 61.84; | H, 5.14; | N, 10.28 |

Procedure 10 Cyclohexanecarboxylic acid [1-(2-oxo-2H-1-benzopyran-3-yl)ethylidene]hydrazide. Compound 251.

A mixture of 4.0 gm (0.0213 mole) of 3-acetylcoumarin, 3.03 gm (0.0213 mole) of cyclohexamecarboxylic acid hydrazide, 100 ml of ethanol and 10 drops of glacial acetic acid is refluxed 8 hr. The reaction mixture is chilled. The product is collected, washed with water and dried to give 4.62 gm (69%) of the title compound having a melting point of 190.5° C.

| | | | |
|---|---|---|---|
| Analysis Calcd: | C,69.22; | H, 6.45; | N, 8.96 |
| Found: | C, 68.94; | H, 6.32; | N, 8.83 |

The compounds prepared according to Procedures 1-10 are tabulated in Table A along with other illustrative compounds of the invention prepared following the general procedure indicated (Procedures 1-10) and making non-critical variations, except starting with the appropriate ketone/aldehyde (II) and

acylhydrazide/carbazate (III).

The acylhydrazones of this invention (Formula I) are effective against parasitic worms, particularly those of valuable domestic warm-blooded animals and more particularly helminth parasites in ovines (sheep) and bovines (cattle).

Observations in sheep experimentally infected with Haemonchus contortus in accordance with Procedure A, generally confirm anthelmintic activity at 100 mg/kg of body weight upon oral and/or parenteral administration as set forth in Table I. Acylhydrazones which are toxic at 100 mg/kg are expected to exhibit anthelmintic activity at a lower non-toxic dose. Further observations in sheep naturally infected with various helminths also confirmed broad-spectrum anthelmintic activity of acylhydrazones of this invention. See Procedure B and the results as set forth in Tables IIA and IIB.

Procedure No. A

In individual experiments all sheep are treated identically, however non-critical variations occur between experiments. All of the sheep used in this procedure are treated twice with levamisole hydrochloride orally at 8 mg/kg or once each with ivermectin parenterally at 200 µg/kg and levamisole hydrochloride orally at 8 mg/kg. The second treatment in each case is administered 4-7 days after the first treatment. Two weeks after the second treatment all sheep are inoculated per os with ~3,500 to ~7,500 infective larvae of H. contortus. Rectal fecal samples are taken from each sheep 26-41 days post-inoculation (PI), and these samples are examined for eggs of H. contortus using the McMaster counting chamber technique. All sheep harboring good infections of H. contortus are randomly allocated to a treatment group; those which do not exhibit suitable infections are dropped from the study. One-three days later on days 27-42 PI each sheep remaining in the study (excluding the nontreated controls) is treated with a test compound (orally or parenterally at 100 mg/kg unless indicated otherwise) or a standard (levamisole hydrochloride orally at 8 mg/kg) or is used as an untreated control. All sheep received food and water ad lib. throughout the experiment.

Prior to administration, all solid compounds are finely ground using a mortar and pestle. Oral compounds are suspended in 20-30 ml of sterile vehicle #98 (each ml contains: carboxymethylcellulose - 10 mg, polysorbate 80-4 mg, propylparaben - 0.42 mg) using a sonicator and administered along with a tap water wash via a stomach tube. The parenteral compounds are similarly suspended in 20-30 ml of the sterile vehicle and given by intraperitoneal injection using a 13 gauge, 2 inch needle and a 50 ml syringe. All test compounds are given to a single sheep/route of administration. Two or more sheep are treated with levamisole hydrochloride and five are used as nontreated controls. All animals are monitored for signs of toxicity following treatment.

The sheep are sacrificed 7-12 days after treatment (days 35-49 PI), and the abomasum is ligated and removed from each sheep. Each abomasum is longitudinally sectioned and rinsed into an 80 mesh sieve. Sieve contents are collected in individual containers and fixed in formol-alcohol. Later each sample is transferred to a 1000 or 2000 ml beaker and the volume brought to 400-1000 ml with tap water. The total number of worms in a 40-100 ml aliquot (10%) is determined. When no worms are found in the 10% aliquot, the entire sample is examined. Total worm number/sheep and percentage clearance for each treatment are calculated. Percentage clearance for a particular test compound in a given trial is determined according to the following formula:

Percentage Clearance = [(Mean number of worms recovered from non-treated control sheep - Number of worms recovered from treated sheep)/Mean number of worms recovered from nontreated control sheep] x 100.

Sheep which die within 24 hr following treatment are not examined for worms (Toxic), while any that die between 24 hr post-treatment and necropsy are examined in an identical manner as that described above. The results of various trials are combined and reported in Table I as percentage clearance.

Procedure No. B

Parasitized sheep are randomly assigned to groups of five animals based on parasitic burden, sex, and farm origin. Sheep are double ear-tagged, weighed, housed in a barn in community pens, fed hay supplemented with 1/2 lb corn/head/day. Water is given ad lib. Animals are allowed to acclimate for one-two weeks prior to treatment.

Each group of sheep receives a test compound either orally or parenterally at a dosage rate of 100

mg/kg. A group of sheep is treated with 8 mg/kg of levamisole hydrochloride and another group serves as an untreated control group. Orally administered compounds are suspended in 20-30 ml of sterile vehicle #98 (each ml contains: carboxymethylcellulose - 10 mg, polysorbate 80-4 mg, propylparaben - 0.42 mg) using a sonicator and administered along with a tap water wash via a stomach tube. For parenteral administration, compounds are similarly suspended in 20-30 ml of the sterile vehicle and given by intraperitoneal injection using a 20 gauge, 1 inch needle and a 50 ml syringe. Following treatment, all animals are observed for signs of toxicity.

The number and classification of helminth eggs per gram of feces (e.p.g.) are determined for each sheep during the acclimation period and in some cases at necropsy. Egg counts are made using the McMaster counting chamber technique and rectal fecal samples. Animals dying during the 24 hours immediately following dosing are not subjected to necropsy. Sheep that die 1-6 days posttreatment are posted and complete worm counts performed. All remaining animals are sacrificed on days 7-8 posttreatment. Each sheep is euthanised and bled out prior to opening the abdominal cavity. Ligatures are placed at the reticulo-omasal junction, the pyloric valve, and the ileocecal junction. The abdomen and small intestine are freed of fat and mesenteric attachments, longitudinally opened, and their contents placed in individual containers. The mucosal surface of each is washed with tap water, rubbed clean, and rinsed several times. Washings and ingesta for each organ are made up to 1 liter and a 10% aliquot in formalin is stored for later examination. The cecum, large intestine, and colon are freed of mesenteric attachments, each is longitudinally opened, and their contents washed, collected, and made up to 1 liter in 10% formalin. The entire sample is stored. All carcasses are incinerated.

Ten percent of the total contents collected from the abomasum and small intestine and the entire contents of the large intestine, cecum, and colon are examined under stereoscopic magnification (40X). All worms are identified to genus and in some instances species. Separate adult and larval counts are determined.

The mean percentage clearance against specific helminths in the test sheep is calculated by subtracting the mean number of helminths observed in the treated sheep at necropsy from the mean number observed in the nontreated controls at necropsy, dividing the remainder by the latter mean number and multiplying by 100. The mean percentage clearances against the various helminths identified in the test sheep are calculated. The results for a acylhydrazone of Formula I are set forth in Tables IIA and IIB.

From an evaluation of the test results set forth in Tables I and IIA and IIB, it is clear that the acylhydrazones of this invention (Formula I) are broad-spectrum anthelmintic agents.

DETAILED DESCRIPTION (cont'd)

The acylhydrazones of Formula I can be used as the pure compounds or as mixtures of pure compounds but for practical reasons the compounds are preferably formulated as anthelmintic compositions and administered as a single or multiple dose, alone or in combination with other anthelmintics (e.g. avermectins, benzimidazoles, levamisole, praziquantel, etc.). For example, aqueous or oil suspensions can be administered orally, or the compounds can be formulated with a solid carrier for feeding. Furthermore, an oil suspension can be converted into an aqueous emulsion by mixing with water and injecting the emulsion intramuscularly, subcutaneously or into the peritoneal cavity. In addition, the active compound(s) can be administered topically to the animal in a conventional pour-on formulation.

Pure compounds, mixtures of the active compounds, or combinations thereof with a solid carrier can be administered in the animal's food, or administered in the form of tablets, pills, boluses, wafers, pastes, and other conventional unit dosage forms, as well as sustained release dosage forms which deliver the active compound over an extended period of days, weeks or months. All of these various forms of the active compounds of this invention can be prepared using physiologically acceptable carriers and known methods of formulation and manufacture.

Representative solid carriers conveniently available and satisfactory for physiologically acceptable, unit dosage formulations include corn starch, powdered lactose, powdered sucrose, talc, stearic acid, magnesium stearate, finely divided bentonite, and the like. The active agent can be mixed with a carrier in varying proportions from, for example, about 0.001 percent by weight in animal feed to about 90 or 95 percent or more in a pill or capsule. In the latter form, one might use no more carrier than sufficient to bind the particles of active compound.

In general, the compounds can be formulated in stable powders or granules for mixing in an amount of feed for a single feeding or enough feed for one day and thus obtain therapeutic efficacy without complication. It is the prepared and stored feeds or feed premixes that require care. A recommended practice is to coat a granular formulation to protect and preserve the active ingredient. A prepared hog-feed

containing about 0.2 percent of the active compound will provide a dosage of about 100 mg per kg body weight for each 100 lb pig in its daily ration.

A solid diluent carrier need not be a homogeneous entity, but mixtures of different diluent carriers can include small proportions of adjuvants such as water; alcohols; protein solutions and suspensions like skimmed milk; edible oils; solutions, e.g., syrups; and organic adjuvants such as propylene glycols, sorbitol, glycerol, diethyl carbonate, and the like.

The solid carrier formulations of the inventions are conveniently prepared in unit dosage forms, to facilitate administration to animals. Accordingly, several large boluses (about 20 g weight) amounting to about 54 g of active compound would be required for a single dosage to a 900 lb horse at a dosage rate of 50 mg/kg of body weight. Similarly, a 60 lb lamb at a dosage rate of 100 mg/kg of body weight would require a pill, capsule, or bolus containing about 2.7 g of active compound. A small dog, on the other hand, weighing about 20 lbs. would require a total dosage of about 225 mg at a dosage rate of 25 mg/kg of body weight. The solid, unit dosage forms can be conveniently prepared in various sizes and concentrations of active ingredient, to accomodate treatment of the various sizes of animals that are parasitized by worms.

Liquid formulations can also be used. Representative liquid formulations include aqueous (including isotonic saline) suspensions, oil solutions and suspensions, and oil in water emulsions. Aqueous suspensions are obtained by dispersing the active compound in water, preferably including a suitable surface-active dispersing agent such as cationic, anionic, or non-ionic surface-active agents. Representative suitable ones are polyoxyalkylene derivatives of fatty alcohols and of sorbitan esters, and glycerol and sorbitan esters of fatty acids. Various dispersing or suspending agents can be included and representative ones are synthetic and natural gums, tragacanth, acacia, alginate, dextran, gelatin, sodium carboxymethylcellulose, methylcellulose, sodium polyvinylpyrrolidone, and the like. The proportion of the active compound in the aqueous suspensions of the invention can vary from about 1 percent to about 20 percent or more.

Oil solutions are prepared by mixing the active compound and an oil, e.g. an edible oil such as cottonseed oil, peanut oil, coconut oil, modified soybean oil, and sesame oil. Usually, solubility in oil will be limited and oil suspensions can be prepared by mixing additional finely divided compound in the oil.

Oil in water emulsions are prepared by mixing and dispersing an oil solution or suspension of the active compound in water preferably aided by surface-active agents and dispersing or suspending agents as indicated above.

In general, the formulations of this invention are administered to animals so as to achieve therapeutic or prophylactic levels of the active compound. At present, it is known that a dose of 100 mg/kg of body weight in sheep of a acylhydrazone of this invention will effectively combat a wide variety of parasites. Much lower effective dosages of various compounds are contemplated, e.g., in the range of 1 to 75 mg/kg of body weight.

In other animals, and for other kinds of parasitic worms, definitive dosages can be proposed. Contemplated are dosage rates of about 1 mg to about 800 mg/kg of body weight. A preferred, contemplated range of dosage rates is from about 5 mg to about 400 mg/kg of body weight. In this regard, it should be noted that the concentration of active compound in the formulation selected for administration is in many situations not critical. One can administer a larger quantity of a formulation having a relatively low concentration and achieve the same therapeutic or prophylactic dosage as a relatively small quantity of a relatively more concentrated formulation. More frequent small dosages will likewise give results comparable to one large dose. One can also administer a sustained release dosage system (protracted delivery formulation) so as to provide therapeutic and/or prophylactic dosage amounts over an extended period. Unit dosage forms in accordance with this invention can have anywhere from less than 1 mg to 500 g of active compound per unit.

Although the anthelmintic agents of this invention will find their primary use in the treatment and/or prevention of helminth parasitisms in domesticated animals such as sheep, cattle, horses, dogs, swine, goats and poultry, they are also effective in treatment that occurs in other warm blooded animals including man. The optimum amount to be employed for best results will, of course, depend upon the particular compound employed, species of animal to be treated, the regimen treatment and the type and severity of helminth infection. Generally good results are obtained with compounds of Formula I by the oral or parenteral route of administration of about 1 to 300 mg/kg of animal bodyweight (such total dose being given at one time, in a protracted manner or in divided doses over a short period of time such as 1-4 days). The technique for administering these materials to animals are known to those skilled in the veterinary and medical fields.

It is contemplated that the acylhydrazones of Formula I can be used to treat various helminth diseases in humans, including those caused by Ascaris, Enterobius, Ancylostoma, Trichuris, Strongyloides, Fasciola, Taenia, and/or Onchocerca or other filaria at a dose of from 1 mg/kg to 300 mg/kg of body weight upon oral

and/or parenteral administration.

DEFINITIONS

The definitions and explanations below are for the terms as used throughout the entire patent application including both the specification and claims.

All temperatures are in degrees Celsius.

TLC refers to thin-layer chromatography.

When solvent pairs are used, the ratio of solvents used are volume/volume (v/v).

TABLE A

| C | Y | a | $R_1$ | $R_2$ | $R_3$ | X | m.p.(°C) | P | H |
|---|---|---|---|---|---|---|---|---|---|
| 1 | NH | 3 | H | H | H | N-morpholinyl $CH_2CH_2$ | 235.2 | 1 | + |
| 2 | NH | 3 | H | H | H | 3-ClPh | 224.3 | 2 | - |
| 3 | NH | 3 | H | H | H | $CH_3CH_2$ | 247.5 | 2 | - |
| 4 | NH | 3 | H | H | H | t-$C_4H_9O$ | 136.2 | 3 | - |
| 5 | NH | 3 | H | H | H | 2-thienyl | 272.7 | 2 | - |
| 6 | NH | 3 | H | H | H | 4-$CH_3CH_2OPh$ | 234.6 | 1 | - |
| 7 | NH | 3 | H | H | H | 1-menthylO | 222.2d | 1 | - |
| 8 | 0 | 2 | H | H | $CH_3$ | $CH_3CH_2O$ | 167.8 | 4 | - |
| 9 | 0 | 2 | H | H | $CH_3$ | t-$C_4H_9O$ | 176.7 | 4 | - |
| 10 | 0 | 2 | H | H | $CH_3$ | $PhCH_2O$ | 251.0 | 4 | |
| 11 | 0 | 2 | H | H | $CH_3$ | $Ph_2CHO$ | 222.3 | 4 | - |
| 12 | NH | 3 | H | H | H | $CH_3CH_2O$ | 147.1 | 2 | - |
| 13 | NH | 3 | H | H | H | 3-pyridinyl | 230.8 | 2 | - |
| 14 | NH | 3 | H | H | H | N-morpholinyl-$CH_2(CH_3)CH$ | 214.8 | 2 | - |
| 15 | NH | 3 | H | H | H | 2-ClPh | 231.4 | 5 | |
| 16 | NH | 3 | H | H | H | 4-ClPh | 230.5 | 5 | - |
| 17 | NH | 3 | H | H | H | 4-$NO_2Ph$ | 247.0 | 5 | - |
| 18 | NH | 3 | H | H | H | 2-HOPh | 256.0 | 5 | + |
| 19 | NH | 3 | H | H | H | 3-$NO_2Ph$ | 101.7 | 5 | - |
| 20 | NH | 3 | H | H | H | $CNCH_2$ | 176.2 | 5 | - |
| 21 | NH | 3 | H | H | H | 3-pyridinyl | 230.3 | 5 | - |
| 22 | NH | 3 | H | H | H | 2-furanyl | 276.0 | 5 | - |
| 3 | NH | 3 | H | H | H | 3,5-$(NO_2)_2Ph$ | 285.2 | 5 | - |
| 24 | 0 | 2 | H | H | $CH_3$ | H | 185.6 | 3 | - |
| 25 | 0 | 2 | H | H | $CH_3$ | $CH_3$ | 178.0 | 3 | - |
| 26 | 0 | 2 | H | H | $CH_3$ | $CH_3CH_2$ | 123.9 | 3 | - |
| 27 | 0 | 2 | H | H | $CH_3$ | $CH_3CH_2CH_2$ | 160.1 | 3 | - |
| 28 | 0 | 2 | H | H | $CH_3$ | i-$C_3H_2$ | 139.6 | 3 | - |
| 29 | 0 | 2 | H | H | $CH_3$ | c-$C_3H_5$ | 223.3 | 3 | - |
| 30 | 0 | 2 | H | H | $CH_3$ | PhO | 176.0 | 3 | - |
| 31 | NH | 3 | H | H | H | H | 213.7 | 2 | - |
| 32 | NH | 3 | H | H | H | $CH_3O$ | 124.5 | 3 | - |
| 33 | NH | 3 | H | H | H | $PhCH_2O$ | 166.9 | 3 | - |

TABLE A (cont'd)

| C | Y | a | $R_1$ | $R_2$ | $R_3$ | X | m.p.(°C) | P | H |
|---|---|---|---|---|---|---|---|---|---|
| 34 | NH | 3 | H | H | H | Ph | 237.3 | 3 | + |
| 35 | NH | 3 | H | H | H | $CH_3CH_2CH_2$ | 227.0 | 3 | |
| 36 | NH | 3 | H | H | H | $PhCH_2$ | 209.9 | 3 | - |
| 37 | NH | 3 | H | H | H | 2-$CH_3$Ph | 273.9 | 3 | - |
| 38 | NH | 3 | H | H | H | 3,5-$(CH_3O)_2$Ph | 244.2 | 5 | - |
| 39 | NH | 3 | H | H | H | 2-$CH_3$OPh | 144.8 | 3 | + |
| 40 | NH | 3 | H | H | H | 3-$CH_3$OPh | >300° | 3 | - |
| 41 | NH | 3 | H | H | H | 4-$CH_3$OPh | 255.9 | 5 | - |
| 42 | NH | 3 | H | H | H | 2-PhOPh | 226.8 | 5 | - |
| 43 | NH | 3 | H | H | H | 1-naphthyl | 300°d | 5 | + |
| 44 | NH | 3 | H | H | H | 4-PhPh | 255.2 | 5 | + |
| 45 | NH | 3 | H | H | H | 3,4-$(CH_3O)_2$Ph | 206.0 | 5 | - |
| 46 | NH | 3 | H | H | H | 2,5-$(CH_3O)_2$Ph | 204.3 | 5 | - |
| 47 | NH | 3 | H | H | $CH_3$ | 2-HOPh | 249.7 | 5 | - |
| 48 | NH | 3 | H | H | $CH_3$ | Ph | 232.3 | 5 | + |
| 49 | $NCH_2Ph$ | 3 | H | H | $CH_3CH_2$ | $CH_3CH_2CH_2$ | 177.4 | 6 | - |
| 50 | NH | 3 | 5-Br | H | H | $CH_3CH_2CH_2$ | 256.4 | 2 | - |
| 51 | NH | 3 | H | H | H | 4-pyridinyl | 160.8 | 3 | - |
| 52 | NH | 3 | 5-Br | H | H | $CH_3CH_2O$ | 177.8 | 3 | - |
| 53 | NH | 3 | 5-Br | H | H | Ph | 241.7d | 3 | - |
| 54 | NH | 3 | 5-Br | H | H | 4-pyridinyl | >300 | 2 | - |
| 55 | NH | 3 | 5-Br | H | H | c-$C_6H_{11}$ | 244.9 | 2 | - |
| 56 | $NCH_2Ph$ | 3 | H | H | $CH_3CH_2$ | $CH_3CH_2O$ | 102.5 | 2 | - |
| 57 | $NCH_2Ph$ | 3 | H | H | $CH_3CH_2$ | Ph | 175.0 | 2 | - |
| 58 | $NCH_2Ph$ | 3 | H | H | $CH_3CH_2$ | 4-pyridinyl | 200.8 | 7 | - |
| 59 | $NCH_2Ph$ | 3 | H | H | $CH_3CH_2$ | c-$C_6H_{11}CH_2$ | 180.5 | 7 | - |
| 60 | $NCH_2Ph$ | 3 | H | H | $CH_3CH_2$ | c-$C_4H_7$ | 185.7 | 7 | - |
| 61 | $NCH_2Ph$ | 3 | H | H | $CH_3CH_2$ | H | 195.2 | 7 | - |
| 62 | $NCH_2Ph$ | 3 | H | H | $CH_3CH_2$ | 4-ClPh | 138.8 | 2 | - |
| 63 | $NCH_2Ph$ | 3 | H | H | $CH_3CH_2$ | 4-$CH_3$Ph | 158.7 | 2 | - |
| 64 | $NCH_2Ph$ | 3 | H | H | $CH_3CH_2$ | $CH_3$ | 195.9 | 2 | - |
| 65 | NH | 3 | H | H | H | 4-$CH_3$Ph | >300 | 2 | + |
| 66 | NH | 3 | H | H | H | c-$C_4H_7$ | 254.0d | 2 | + |
| 67 | NH | 3 | H | 2-$CH_3$ | H | $CH_3CH_2O$ | 138.8 | 7 | + |
| 68 | NH | 3 | H | 2-$CH_3$ | H | 4-pyridinyl | >300 | 2 | + |

TABLE A (cont'd)

| C | Y | a | R1 | R2 | R3 | X | m.p.(°C) | P | H |
|---|---|---|---|---|---|---|---|---|---|
| 69 | NH | 3 | H | 2-$CH_3$ | H | $CH_3CH_2CH_2$ | 165.5 | 2 | + |
| 70 | NH | 3 | H | 2-$CH_3$ | H | $CH_3CH_2$ | 176.0 | 2 | + |
| 71 | NH | 3 | H | 2-$CH_3$ | H | c-$C_3H_5$ | 117.2 | 2 | + |
| 72 | NH | 3 | H | 2-$CH_3$ | H | i-$C_3H_7$ | 202.2 | 2 | + |
| 73 | NH | 3 | H | H | H | $CH_3$ | 261.4 | 7 | - |
| 74 | NH | 3 | H | H | H | c-$C_6H_{11}CH_2$ | 249.3 | 7 | - |
| 75 | NH | 3 | H | H | H | c-$C_5H_{11}CH_2CH_2$ | 228.8 | 7 | + |
| 76 | NH | 3 | H | H | H | c-$C_6H_{11}CH_2CH_2$ | 229.7 | 7 | - |
| 77 | NH | 3 | H | H | H | i-$C_3H_7$ | >300 | 7 | - |
| 78 | NH | 3 | H | H | $CH_3$ | $CH_3CH_2CH_2$ | 166.5 | 7 | - |
| 79 | NH | 3 | H | H | $CH_3$ | $CH_3CH_2O$ | 140.7 | 7 | - |
| 80 | NH | 3 | H | H | $CH_3$ | 4-pyridinyl | 246.9 | 7 | - |
| 81 | O | 2 | H | H | $CH_3$ | H | 185.6 | 3 | - |
| 82 | O | 2 | H | H | $CH_3$ | $CH_3$ | 178.0 | 3 | - |
| 83 | O | 2 | H | H | $CH_3$ | $CH_3CH_2$ | 123.9 | 3 | - |
| 84 | O | 2 | H | H | $CH_3$ | $CH_3CH_2CH_2$ | 160.1 | 3 | - |
| 85 | O | 2 | H | H | $CH_3$ | i-$C_3H_7$ | 139.6 | 3 | - |
| 86 | O | 2 | H | H | $CH_3$ | c-$C_3H_5$ | 223.3 | 3 | - |
| 87 | NH | 3 | H | H | H | c-$C_6H_{11}$ | 220.2 | 7 | + |
| 88 | NH | 3 | H | 2-$CH_3$ | H | c-$C_6H_{11}$ | 223.6 | 7 | - |
| 89 | NH | 3 | H | 2-$CH_3$ | H | 4-t-$C_4H_9$Ph | 269.9 | 7 | + |
| 90 | NH | 3 | H | 2-$CH_3$ | H | 4-$CF_3$Ph | 210.8 | 7 | - |
| 91 | NH | 3 | H | 2-$CH_3$ | H | 3,4-$(CH_3O)_2PhCH_2$ | 119.7 | 7 | + |
| 92 | NH | 3 | H | 2-$CH_3$ | H | $PhCH_2O$ | 168.3 | 7 | - |
| 93 | NH | 3 | H | 2-$CH_3$ | H | 4-$CH_3$Ph | 260.1 | 7 | - |
| 94 | NH | 3 | H | 2-$CH_3$ | H | 2-ClPh | 211.6 | 7 | + |
| 95 | NH | 3 | H | 2-$CH_3$ | H | 4-ClPh | 284.6 | 7 | + |
| 96 | NH | 3 | H | 2-$CH_3$ | H | 3-pyridinyl | 271.5 | 7 | + |
| 97 | NH | 3 | H | 2-$CH_3$ | H | t-$C_4H_9O$ | 131.8 | 7 | + |
| 98 | NH | 3 | H | 2-$CH_3$ | H | 4-$CH_3OPh$ | 227.1 | 7 | + |
| 99 | NH | 3 | 5-Br | H | H | i-$C_3H_7$ | 230.4 | 3 | - |
| 100 | NH | 3 | 5-Br | H | H | 4-t-$C_4H_9$Ph | 260.7 | 3 | - |
| 101 | O | 2 | H | H | $CH_3$ | $CH_3O$ | 154.4 | 2 | - |
| 102 | O | 2 | H | H | $CH_3$ | 1-menthyl | 189.7 | 3 | - |
| 103 | O | 2 | H | H | $CH_3$ | c-$C_6H_{11}$ | 209.0 | 3 | - |

TABLE A (cont'd)

| C | y | a | $R_1$ | $R_2$ | $R_3$ | X | m.p.(°C) | P | H |
|---|---|---|---|---|---|---|---|---|---|
| 104 | O | 2 | H | H | $CH_3$ | c-$C_6H_{11}CH_2$ | 165.0 | 3 | - |
| 105 | O | 2 | H | H | $CH_3$ | c-$C_6H_{11}CH_2CH_2$ | 153.2 | 3 | - |
| 106 | O | 2 | H | H | $CH_3$ | $CH_3CH_2OCH_2CH_2$ | 109.0 | 3 | - |
| 107 | O | 2 | H | H | $CH_3$ | chrysanthemyl | 154.2 | 3 | - |
| 108 | O | 2 | H | H | $CH_3$ | $PhCH_2$ | 200.0 | 2 | - |
| 109 | O | 2 | H | H | $CH_3$ | 4-$CH_3OPhCH_2O$ | 171.5 | 3 | - |
| 110 | O | 2 | H | H | $CH_3$ | Ph | 164.0 | 3 | - |
| 111 | O | 2 | H | H | $CH_3$ | 2-ClPh | 160.6 | 3 | - |
| 112 | O | 2 | H | H | $CH_3$ | 2-$CH_3OPh$ | 131.9 | 3 | - |
| 113 | O | 2 | H | H | $CH_3$ | 3-CH3OPh | 146.9 | 3 | - |
| 114 | O | 2 | H | H | $CH_3$ | 4-$CH_3OPh$ | 194.1 | 3 | - |
| 115 | O | 2 | H | H | $CH_3$ | 2-$CH_3Ph$ | 159.5 | 3 | - |
| 116 | O | 2 | H | H | $CH_3$ | 3-$CH_3Ph$ | 160.0 | 3 | - |
| 117 | O | 2 | H | H | $CH_3$ | 4-$CH_3Ph$ | 210.5 | 3 | - |
| 118 | S | 3 | H | H | $CH_3$ | $CH_3$CH2$CH_2$ | 146.5 | 7 | - |
| 119 | S | 3 | H | H | $CH_3$ | $CH_3CH_2O$ | 135.5 | 6 | - |
| 120 | S | 3 | H | H | $CH_3$ | Ph | 185.2 | 6 | - |
| 121 | S | 3 | H | H | $CH_3$ | 4-pyridinyl | 239.3 | 7 | - |
| 122 | S | 3 | H | H | $CH_3$ | c-$C_6H_{11}$ | 206.1 | 2 | - |
| 123 | S | 3 | H | H | $CH_3$ | $CH_3CH_2$ | 155.6 | 7 | - |
| 124 | S | 3 | H | H | $CH_3$ | c-$C_6H_{11}CH_2CH_2$ | 157.7 | 7 | - |
| 125 | S | 3 | H | H | $CH_3$ | $PhCH_2O$ | 149.5 | 6 | - |
| 126 | S | 3 | H | H | $CH_3$ | 2-ClPh | 197.1 | 6 | - |
| 127 | S | 3 | H | H | $CH_3$ | 3-ClPh | 190.2 | 6 | - |
| 128 | S | 3 | H | H | $CH_3$ | 4-ClPh | 186.3 | 6 | - |
| 129 | S | 3 | H | H | $CH_3$ | 1-menthyl O | 152.3 | 6 | - |
| 130 | S | 3 | H | H | $CH_3$ | 4-$CH_3Ph$ | 175.7 | 6 | - |
| 131 | S | 3 | H | H | $CH_3$ | 4-$CH_3CH_2OPh$ | 163.4 | 6 | - |
| 132 | S | 3 | H | H | $CH_3$ | 3,5-$(CH_3O)_2Ph$ | 167.7 | 6 | - |
| 133 | S | 3 | H | H | $CH_3$ | 4-$CF_3Ph$ | 184.3 | 6 | - |
| 134 | S | 3 | H | H | $CH_3$ | 4-t-$C_4H_9Ph$ | 162.4 | 6 | - |
| 135 | S | 3 | H | H | $CH_3$ | 4-$(CH_3)_2NPh$ | 213.2 | 7 | - |
| 136 | S | 3 | H | H | $CH_3$ | 2-furanyl | 154.4 | 6 | - |
| 137 | S | 3 | H | H | $CH_3$ | 2-thienyl | 172.6 | 6 | - |
| 138 | NH | 3 | 2-$CH_3$ | H | H | Ph | 224.2 | 7 | + |
| 139 | NH | 3 | H | H | H | 4-t-$C_4H_9Ph$ | 265.9 | 2 | - |

TABLE A (cont'd)

| C | Y | a | $R_1$ | $R_2$ | $R_3$ | X | m.p.(°C) | P | H |
|---|---|---|---|---|---|---|---|---|---|
| 140 | NH | 3 | H | H | H | $c\text{-}C_3H_5$ | 272.5 | 2 | - |
| 141 | NH | 3 | H | H | H | $3\text{-}CH_3Ph$ | 213.0 | 2 | - |
| 142 | NH | 3 | H | H | H | $2\text{-}CH_3CH_2OPh$ | 209.1 | 2 | - |
| 143 | NH | 3 | H | H | H | 1-naphthyl$CH_2$ | 236.3 | 2 | - |
| 144 | NH | 3 | H | H | H | $4\text{-}CH_3OPhCH_2O$ | 178.8 | 2 | - |
| 145 | $NCOCH_3$ | 3 | H | H | H | $n\text{-}C_3H_7$ | 199.8 | 2 | - |
| 146 | $NCOCH_3$ | 3 | H | H | H | $CH_3CH_2O$ | 241.0 | 2 | - |
| 147 | $NCOCH_3$ | 3 | H | H | H | Ph | 236.9 | 2 | - |
| 148 | $NCOCH_3$ | 3 | H | H | H | 4-pyridinyl | 250.1 | 2 | - |
| 149 | $NCOCH_3$ | 3 | H | H | H | $c\text{-}C_6H_{11}$ | 271.1 | 2 | - |
| 150 | $NCOCH_3$ | 3 | H | H | H | $c\text{-}C_3H_7$ | 260.2 | 2 | - |
| 151 | $NCOCH_3$ | 3 | H | H | H | $i\text{-}C_3H_7$ | 227.6 | 2 | - |
| 152 | $NCOCH_3$ | 3 | H | H | H | $c\text{-}C_4H_7$ | 239.7 | 2 | - |
| 153 | $NCOCH_3$ | 3 | H | H | H | $t\text{-}C_4H_9O$ | 215.2 | 2 | - |
| 154 | $NCOCH_3$ | 3 | H | H | H | $4\text{-}CH_3Ph$ | 250.0 | 2 | - |
| 155 | $NCOCH_3$ | 3 | H | H | H | $3\text{-}CH_3Ph$ | 251.9 | 2 | - |
| 156 | $NCOCH_3$ | 3 | H | H | H | 1-naphthyl$CH_2$ | 276.1 | 2 | - |
| 157 | $NCOCH_3$ | 3 | H | H | H | $2\text{-}CH_3CH_2OPh$ | 201.6 | 2 | - |
| 158 | $NCOCH_3$ | 3 | H | H | H | $4\text{-}CH_3CH_2OPh$ | 232.7 | 2 | - |
| 159 | $NCOCH_3$ | 3 | H | H | H | $4\text{-}t\text{-}C_4H_9Ph$ | 256.3 | 2 | - |
| 160 | $NCOCH_3$ | 3 | H | H | H | $3,4\text{-}(CH_3O)_2PhCH_2$ | 186.7 | 2 | - |
| 161 | NH | 3 | H | $2\text{-}CH_3$ | H | $CH_3O$ | 152.7 | 8 | + |
| 162 | NH | 3 | H | $2\text{-}CH_3$ | H | $2,5\text{-}(Cl)_2Ph$ | 223.6 | 8 | + |
| 163 | NH | 3 | H | $2\text{-}CH_3$ | H | $4\text{-}CH_3CH_2OPh$ | 221.0 | 2 | + |
| 164 | NH | 3 | H | $2\text{-}CH_3$ | H | $CH_3$ | >300 | 2 | + |
| 165 | NH | 3 | H | $2\text{-}CH_3$ | H | $c\text{-}C_6H_{11}CH_2CH_2$ | 182.1 | 8 | + |
| 166 | NH | 3 | H | $2\text{-}CH_3$ | H | $c\text{-}C_6H_{11}CH_2$ | 174.7 | 8 | + |
| 167 | NH | 3 | H | $2\text{-}CH_3$ | H | $PhCH_2$ | 188.5 | 2 | - |
| 168 | NH | 3 | H | $2\text{-}CH_3$ | H | $c\text{-}C_4H_7$ | 173.8 | 8 | + |
| 169 | NH | 3 | H | $2\text{-}CH_3$ | H | $3\text{-}CH_3Ph$ | >300 | 8 | + |
| 170 | NH | 3 | H | $2\text{-}CH_3$ | H | $2\text{-}CH_3Ph$ | 160.4 | 8 | + |
| 171 | NH | 3 | H | $2\text{-}CH_3$ | H | 2-PhOPh | 217.8 | 2 | + |
| 172 | NH | 3 | H | $2\text{-}CH_3$ | H | $2\text{-}CH_3OPh$ | >300 | 8 | + |
| 173 | $NCOCH_3$ | 3 | H | H | H | $CH_3O$ | 231.4 | 2 | - |
| 174 | $NCOCH_3$ | 3 | H | H | H | $2,5\text{-}(Cl)_2Ph$ | 221.9 | 2 | - |
| 175 | $NCOCH_3$ | 3 | H | H | H | 3-pyridinyl | 229.3 | 2 | - |

TABLE A (cont'd)

| C | Y | a | $R_1$ | $R_2$ | $R_3$ | X | m.p. (°C) | P | H |
|---|---|---|---|---|---|---|---|---|---|
| 176 | $NCOCH_3$ | 3 | H | H | H | 4-ClPh | 253.4 | 2 | - |
| 177 | $NCOCH_3$ | 3 | H | H | H | 2-ClPh | 222.3 | 2 | - |
| 178 | $NCOCH_3$ | 3 | H | H | H | 3-ClPh | 228.2 | 2 | - |
| 179 | $NCOCH_3$ | 3 | H | H | H | $c-C_6H_{11}CH_2CH_2$ | 224.4 | 2 | - |
| 180 | $NCOCH_3$ | 3 | H | H | H | $4-CH_3OPh$ | 228.7 | 2 | - |
| 181 | $NCOCH_3$ | 3 | H | H | H | $PhCH_2$ | 232.4 | 2 | - |
| 182 | $NCOCH_3$ | 3 | H | H | H | $4-CF_3Ph$ | 258.0d | 2 | - |
| 183 | $NCOCH_3$ | 3 | H | H | H | $2-CH_3Ph$ | 244.3 | 2 | - |
| 184 | $NCOCH_3$ | 3 | H | H | H | 2-PhOPh | 216.5 | 2 | - |
| 185 | $NCOCH_3$ | 3 | H | H | H | $2-CH_3OPh$ | 200.9 | 2 | + |
| 186 | $NCOCH_3$ | 3 | H | H | H | $4-CH_3OPhCH_2O$ | 236.6 | 2 | - |
| 187 | $NCOCH_3$ | 3 | H | H | H | $PhCH_2O$ | 248.0 | 2 | - |
| 188 | $NCOCH_3$ | 3 | H | H | H | 2-thienyl | 245.5 | 2 | - |
| 189 | $NCOCH_3$ | 3 | H | H | H | 2-furanyl | 221.4 | 2 | + |
| 190 | $NCOCH_3$ | 3 | H | H | H | $3-CH_3OPh$ | 210.9 | 2 | - |
| 191 | $NCOCH_3$ | 3 | H | H | H | $3,5-(CH_3O)_2Ph$ | 239.7 | 2 | - |
| 192 | $NCOCH_3$ | 3 | H | H | H | $2,5-(CH_3O)_2Ph$ | 212.0 | 2 | - |
| 193 | $NCOCH_3$ | 3 | H | H | H | $4-(CH_3)_2NPh$ | 265.9 | 2 | - |
| 194 | NH | 3 | $5-PhCH_2O$ | H | H | $n-C_3H_7$ | 215.2 | 8 | - |
| 195 | NH | 3 | $5-PhCH_2O$ | H | H | $CH_3CH_2O$ | 104.5 | 8 | + |
| 196 | NH | 3 | $5-PhCH_2O$ | H | H | Ph | 257.3 | 5 | + |
| 197 | NH | 3 | $5-PhCH_2O$ | H | H | 4-pyridinyl | 290.7 | 5 | + |
| 198 | NH | 3 | $5-PhCH_2O$ | H | H | $c-C_3H_5$ | 285.1 | 5 | - |
| 199 | NH | 3 | $5-PhCH_2O$ | H | H | $i-C_3H_7$ | >300 | 5 | - |
| 200 | NH | 3 | $5-PhCH_2O$ | H | H | $1-naphthylCH_2$ | 120.3 | 5 | + |
| 201 | NH | 3 | H | $2-CH_3$ | H | $1-naphthylCH_2$ | 233.9 | 2 | + |
| 202 | NH | 3 | H | $2-CH_3$ | H | $3-CH_3OPh$ | 202.3 | 2 | + |
| 203 | NH | 3 | H | $2-CH_3$ | H | $3,5-(CH_3O)_2Ph$ | >300 | 2 | + |
| 204 | NH | 3 | H | $2-CH_3$ | H | $3-CH_3CH_2OPh$ | 249.4 | 2 | + |
| 205 | NH | 3 | H | $2-CH_3$ | H | $3,4-(CH_3O)_2Ph$ | 291.2 | 2 | + |
| 206 | $NCOCH_3$ | 3 | H | H | H | $3-CH_3CH_2OPh$ | 173.1 | 2 | + |
| 207 | NH | 3 | H | H | H | $4-CF_3Ph$ | 225.8 | 2 | + |
| 208 | NH | 3 | H | $5-PhCH_2O$ | H | $c-C_6H_{11}$ | >300 | 5 | + |
| 209 | NH | 3 | H | $2-CH_3$ | H | $4-(CH_3)_2NPh$ | 182.0 | 2 | + |
| 210 | NH | 3 | H | $2-CH_3$ | H | $4-CH_3OPhCH_2O$ | 140.8 | 8 | - |
| 211 | NH | 3 | H | $2-CH_3$ | H | 2-thienyl | 245.4 | 2 | + |

TABLE A (cont'd)

| C | Y | a | R1 | R2 | R3 | X | m.p.(°C) | P | H |
|---|---|---|---|---|---|---|---|---|---|
| 212 | NH | 3 | H | 2-$CH_3$ | H | 2-furanyl | 133.4 | 2 | + |
| 213 | NH | 3 | H | 2-$CH_3$ | H | 2,5-$(CH_3O)_2Ph$ | 189.9 | 2 | + |
| 214 | $NCOCH_3$ | 3 | H | H | H | 3,4,5-$(CH_3O)_3Ph$ | 237.2 | 2 | - |
| 215 | $NCOCH_3$ | 3 | H | H | H | 3,4-$(CH_3O)_2Ph$ | 217.4 | 2 | - |
| 216 | $NCOCH_3$ | 3 | H | H | H | $CH_3CH_2OCH_2CH_2$ | 177.5 | 2 | - |
| 217 | $NCOCH_3$ | 3 | H | H | H | 4-$H_2NPh$ | 269.7 | 2 | - |
| 218 | $NCOCH_3$ | 3 | H | H | H | $CH_3CH_2$ | 224.6 | 2 | - |
| 219 | $NCOCH_3$ | 3 | H | H | H | $CH_3$ | 269.0 | 2 | - |
| 220 | NH | 3 | H | H | H | $CH_3CH_2OCH_2CH_2$ | 206.4 | 2 | - |
| 221 | NH | 3 | H | H | H | 4-$H_2NPh$ | 290.9 | 2 | - |
| 222 | O | 3 | H | H | $CH_3$ | 1-naphthyl$CH_2$ | 235.2 | 8 | - |
| 223 | O | 3 | H | H | $CH_3$ | 3-ClPh | 186.4 | 8 | - |
| 224 | O | 3 | H | H | $CH_3$ | 4-ClPh | 219.8 | 8 | - |
| 225 | O | 2 | H | H | $CH_3$ | 2-PhOPh | 170.1 | 8 | - |
| 226 | O | 2 | H | H | $CH_3$ | 2-$CH_3CH_2OPh$ | 169.8 | 8 | - |
| 227 | O | 2 | H | H | $CH_3$ | 4-$CH_3CH_2OPh$ | 212.3 | 8 | - |
| 228 | O | 2 | H | H | $CH_3$ | 4-t-$C_4H_9Ph$ | 214.0 | 8 | - |
| 229 | O | 2 | H | H | $CH_3$ | 4-$CF_3Ph$ | 212.8 | 2 | - |
| 230 | O | 2 | H | H | $CH_3$ | 4-$(CH_3)_2NPh$ | 225.1 | 2 | - |
| 231 | O | 2 | H | H | $CH_3$ | 2,5-$(CH_3O)_2Ph$ | 185.6 | 2 | - |
| 232 | O | 2 | H | H | $CH_3$ | 3,4-$(CH_3))_2Ph$ | 162.4 | 2 | - |
| 233 | O | 2 | H | H | $CH_3$ | 2-furanyl | 195.9 | 2 | - |
| 234 | O | 2 | H | H | $CH_3$ | 3-pyridinyl | 172.0 | 2 | - |
| 235 | O | 2 | H | H | $CH_3$ | 2,5-$(Cl)_2Ph$ | 190.3 | 2 | - |
| 236 | O | 2 | H | H | $CH_3$ | 3,5-$(CH_3O)_2Ph$ | 196.0 | 2 | - |
| 237 | O | 2 | H | H | $CH_3$ | 2-thienyl | 224.9 | 2 | - |
| 238 | O | 2 | H | H | $CH_3$ | 4-pyridinyl | 239.6 | 2 | - |
| 239 | O | 2 | H | H | $CH_3$ | 3,4,5-$(CH_3O)_3Ph$ | 186.9 | 2 | - |
| 240 | O | 2 | 5-$NO_2$ | H | $CH_3$ | $CH_3CH_2O$ | 208.1 | 2 | - |
| 241 | O | 2 | 5-$NO_2$ | H | $CH_3$ | c-$C_6H_{11}CH_2CH_2$ | 179.0 | 2 | - |
| 242 | O | 2 | 5-$NO_2$ | H | $CH_3$ | c-$C_6H_{11}$ | 245.9 | 2 | - |
| 243 | O | 2 | 5-$NO_2$ | H | $CH_3$ | $CH_3CH_2$ | 238.6 | 2 | - |
| 244 | O | 2 | 5-$NO_2$ | H | $CH_3$ | PhO | 177.6 | 2 | - |
| 245 | O | 2 | 5-$NO_2$ | H | $CH_3$ | $PhCH_2O$ | 202.6 | 2 | - |
| 247 | NH | 3 | H | 2-$CH_3$ | H | 3-ClPh | 219.5 | 8 | - |
| 248 | O | 3 | H | H | $CH_3$ | $OCH_2CH_3$ | 170.2 | 9 | - |

TABLE A (cont'd)

| C | Y | a | $R_1$ | $R_2$ | $R_3$ | X | m.p. (°C) | P | H |
|---|---|---|---|---|---|---|---|---|---|
| 249 | 0 | 3 | H | H | $CH_3$ | $PhCH_2O$ | 176.6 | 9 | - |
| 250 | 0 | 3 | H | H | $CH_3$ | n-$C_3H_7$ | 159.1 | 9 | - |
| 251 | 0 | 3 | H | H | $CH_3$ | c-$C_6H_{11}$ | 190.5 | 10 | - |
| 252 | 0 | 3 | H | H | $CH_3$ | $CH_3CH_2$ | 189.9 | 10 | - |
| 253 | 0 | 3 | H | H | $CH_3$ | c-$C_6H_{11}CH_2CH_2$ | 133.1 | 9 | - |
| 254 | 0 | 3 | H | H | $CH_3$ | 4-pyridinyl | 186.1 | 10 | - |
| 255 | 0 | 3 | H | H | $CH_3$ | 2-ClPh | 120.1 | 10 | + |
| 256 | 0 | 3 | H | H | $CH_3$ | 3-ClPh | 177.2 | 10 | - |
| 257 | 0 | 3 | H | H | $CH_3$ | 4-$CH_3$Ph | 166.3 | 9 | - |
| 258 | 0 | 3 | H | H | $CH_3$ | 4-$CH_3CH_2O$Ph | 177.6 | 10 | + |
| 259 | 0 | 3 | H | H | $CH_3$ | 3,5-$(CH_3O)_2$Ph | 217.7 | 10 | - |
| 260 | 0 | 3 | H | H | $CH_3$ | 4-t-$C_4H_9$Ph | 225.9 | 10 | - |
| 261 | 0 | 3 | H | H | $CH_3$ | 4-$(CH_3)_2N$Ph | 201.0 | 10 | - |
| 262 | 0 | 3 | H | H | $CH_3$ | 2-furanyl | 96.3 | 10 | - |
| 263 | 0 | 3 | H | H | $CH_3$ | 2-thienyl | 215.7 | 10 | - |

Note: n is 0 for compounds #1-247, inclusive;
n is 1 for compounds #248-263, inclusive.

TABLE I

H. Contortus

| Compound No. | % Clearance P.O. | I.P. |
|---|---|---|
| 1 | N.T. | N.T. |
| 2 | 26.5 | N.T. |
| 3 | 73.1 | N.T. |
| 4 | 99.5 | N.T. |
| 5 | 28.3 | N.T. |
| 6 | Toxic | N.T. |
| 7 | N.T. | N.T. |
| 8 | N.T. | N.T. |
| 9 | 0 | N.T. |
| 10 | N.T. | N.T. |
| 11 | 5.8 | 48.6 |
| 12 | 85.5 | N.T. |
| 13 | 87.5 | N.T. |
| 14 | N.T. | N.T. |
| 15 | 48.0 | N.T. |
| 16 | 62.4 | N.T. |
| 17 | N.T. | N.T. |
| 18 | N.T. | N.T. |
| 19 | N.T. | N.T. |
| 20 | 26.9 | N.T. |
| 21 | * | |
| 22 | N.T. | N.T. |
| 23 | N.T. | N.T. |
| 24 | N.T. | N.T. |
| 25 | N.T. | N.T. |
| 26 | N.T. | N.T. |
| 27 | N.T. | N.T. |
| 28 | N.T. | N.T. |
| 29 | N.T. | N.T. |
| 30 | 0 | N.T. |
| 31 | 48.2 | N.T. |
| 32 | 50.0 | N.T. |

TABLE I (cont'd)

H. Contortus

| Compound No. | % Clearance P.O. | I.P. |
|---|---|---|
| 33 | 0 | N.T. |
| 34 | 0 | N.T. |
| 35 | 52.7 | N.T. |
| 36 | 25.5 | N.T. |
| 37 | 40.9 | N.T. |
| 38 | 25.5 | N.T. |
| 39 | N.T. | N.T. |
| 40 | 0+ | N.T. |
| 41 | 22.7 | N.T. |
| 42 | 0 | N.T. |
| 43 | 0 | N.T. |
| 44 | N.T. | N.T. |
| 45 | 1.4 | N.T. |
| 46 | N.T. | N.T. |
| 47 | N.T. | N.T. |
| 48 | 12.7 | 31.0 |
| 49 | 16.8 | N.T. |
| 50 | 49.8 | N.T. |
| 51 | 0 | N.T. |
| 52 | 80.3 | N.T. |
| 53 | 14.7 | N.T. |
| 54 | 0 | N.T. |
| 55 | N.T. | N.T. |
| 56 | 0 | N.T. |
| 57 | 21.1 | N.T. |
| 58 | N.T. | N.T. |
| 59 | 0 | N.T. |
| 60 | N.T. | N.T. |
| 61 | 65.9 | N.T. |
| 62 | 0 | N.T. |
| 63 | N.T. | N.T. |
| 64 | 94.9 | N.T. |

TABLE I (cont'd)

H. Contortus

| Compound No. | % Clearance P.O. | I.P. |
|---|---|---|
| 65 | 0 | N.T. |
| 66 | 86.7 | N.T. |
| 67 | 98.6 | N.T. |
| 68 | N.T. | N.T. |
| 69 | 99.2 | N.T. |
| 70 | 85.7 | N.T. |
| 71 | 0 | N.T. |
| 72 | 52.1 | N.T. |
| 73 | 44.4-64.5 | N.T. |
| 74 | 8.6 | N.T. |
| 75 | N.T. | N.T. |
| 76 | N.T. | N.T. |
| 77 | N.T. | N.T. |
| 78 | 0 | N.T. |
| 79 | 99.5 | N.T. |
| 80 | N.T. | N.T. |
| 81 | 96.0 | N.T. |
| 82 | Toxic | N.T. |
| 83 | 49.6 | N.T. |
| 84 | Toxic | N.T. |
| 85 | * | |
| 86 | * | |
| 87 | N.T. | N.T. |
| 88 | 67.8 | N.T. |
| 89 | 24.5 | N.T. |
| 90 | 50.4 | N.T. |
| 91 | 56.7 | N.T. |
| 92 | 66.7 | N.T. |
| 93 | 39.8 | N.T. |
| 94 | 61.4 | N.T. |
| 95 | 44.2 | N.T. |
| 96 | 97.4 | N.T. |

TABLE I (cont'd)

H. Contortus

| Compound No. | % Clearance P.O. | I.P. |
|---|---|---|
| 97 | 98.2 | N.T. |
| 98 | 46.9 | N.T. |
| 99 | 67.8 | N.T. |
| 100 | N.T. | N.T. |
| 101 | 5.1 | N.T. |
| 102 | N.T. | N.T. |
| 103 | N.T. | N.T. |
| 104 | N.T. | N.T. |
| 105 | N.T. | N.T. |
| 106 | N.T. | N.T. |
| 108 | N.T. | N.T. |
| 109 | N.T. | N.T. |
| 110 | 0 | N.T. |
| 111 | N.T. | N.T. |
| 112 | N.T. | N.T. |
| 113 | N.T. | N.T. |
| 114 | N.T. | N.T. |
| 115 | N.T. | N.T. |
| 116 | N.T. | N.T. |
| 117 | N.T. | N.T. |
| 118 | 93.0 | N.T. |
| 119 | 0 | N.T. |
| 120 | 0 | N.T. |
| 121 | N.T. | N.T. |
| 122 | N.T. | N.T. |
| 123 | 42.1 | N.T. |
| 124 | N.T. | N.T. |
| 125 | N.T. | N.T. |
| 126 | N.T. | N.T. |
| 127 | N.T. | N.T. |
| 128 | 7.6 | N.T. |
| 129 | N.T. | N.T. |
| 130 | N.T. | N.T. |
| 131 | 0 | N.T. |

TABLE I (cont'd)

H. Contortus

| Compound No. | % Clearance P.O. | % Clearance I.P. |
|---|---|---|
| 132 | N.T. | N.T. |
| 133 | N.T. | N.T. |
| 134 | N.T. | N.T. |
| 135 | N.T. | N.T. |
| 136 | N.T. | N.T. |
| 137 | N.T. | N.T. |
| 138 | N.T. | N.T. |
| 139 | N.T. | N.T. |
| 140 | N.T. | N.T. |
| 141 | N.T. | N.T. |
| 142 | N.T. | N.T. |
| 143 | N.T. | N.T. |
| 144 | N.T. | N.T. |
| 145 | 99.7 | N.T. |
| 146 | 0 | N.T. |
| 147 | 66.0 | N.T. |
| 148 | N.T. | N.T. |
| 149 | N.T. | N.T. |
| 150 | N.T. | N.T. |
| 151 | 63.4 | N.T. |
| 152 | N.T. | N.T. |
| 153 | N.T. | N.T. |
| 154 | N.T. | N.T. |
| 155 | N.T. | N.T. |
| 156 | N.T. | N.T. |
| 157 | N.T. | N.T. |
| 158 | 0 | N.T. |
| 159 | N.T. | N.T. |
| 160 | N.T. | N.T. |
| 161 | 50.9 | N.T. |
| 162 | 68.3 | N.T. |
| 163 | 73.9 | N.T. |
| 164 | 78.9 | N.T. |
| 165 | 30.8 | N.T. |

TABLE I (cont'd)

H. Contortus

| Compound No. | % Clearance P.O. | I.P. |
|---|---|---|
| 166 | 65.1 | N.T. |
| 167 | 97.9 | N.T. |
| 168 | N.T. | N.T. |
| 169 | N.T. | N.T. |
| 170 | N.T. | N.T. |
| 171 | N.T. | N.T. |
| 172 | N.T. | N.T. |
| 173 | N.T. | N.T. |
| 174 | N.T. | N.T. |
| 175 | N.T. | N.T. |
| 176 | 0 | N.T. |
| 177 | N.T. | N.T. |
| 178 | N.T. | N.T. |
| 179 | N.T. | N.T. |
| 180 | N.T. | N.T. |
| 181 | N.T. | N.T. |
| 182 | N.T. | N.T. |
| 183 | N.T. | N.T. |
| 184 | N.T. | N.T. |
| 185 | N.T. | N.T. |
| 186 | N.T. | N.T. |
| 187 | N.T. | N.T. |
| 188 | N.T. | N.T. |
| 189 | N.T. | N.T. |
| 190 | N.T. | N.T. |
| 191 | N.T. | N.T. |
| 192 | N.T. | N.T. |
| 193 | N.T. | N.T. |
| 194 | 0 | N.T. |
| 195 | N.T. | N.T. |
| 196 | 63.9 | N.T. |
| 197 | N.T. | N.T. |
| 198 | N.T. | N.T. |
| 199 | 0 | N.T. |

TABLE I (cont'd)

H. Contortus

| Compound No. | % Clearance P.O. | % Clearance I.P. |
|---|---|---|
| 200 | N.T. | N.T. |
| 201 | N.T. | N.T. |
| 202 | N.T. | N.T. |
| 203 | N.T. | N.T. |
| 204 | N.T. | N.T. |
| 205 | N.T. | N.T. |
| 206 | N.T. | N.T. |
| 207 | N.T. | N.T. |
| 208 | N.T. | N.T. |
| 209 | N.T. | N.T. |
| 210 | N.T. | N.T. |
| 211 | N.T. | N.T. |
| 212 | N.T. | N.T. |
| 213 | N.T. | N.T. |
| 214 | N.T. | N.T. |
| 215 | N.T. | N.T. |
| 216 | N.T. | N.T. |
| 217 | N.T. | N.T. |
| 218 | 53.5 | N.T. |
| 219 | 78.9 | N.T. |
| 220 | N.T. | N.T. |
| 221 | N.T. | N.T. |
| 222 | N.T. | N.T. |
| 223 | N.T. | N.T. |
| 224 | 71.8 | N.T. |
| 225 | N.T. | N.T. |
| 226 | N.T. | N.T. |
| 227 | N.T. | N.T. |
| 228 | N.T. | N.T. |
| 229 | N.T. | N.T. |
| 230 | N.T. | N.T. |
| 231 | N.T. | N.T. |
| 232 | N.T. | N.T. |
| 233 | N.T. | N.T. |

TABLE I (cont'd)

H. Contortus

| Compound No. | % Clearance P.O. | % Clearance I.P. |
|---|---|---|
| 244 | N.T. | N.T. |
| 245 | N.T. | N.T. |
| 246 | N.T. | N.T. |
| 247 | 61.5 | N.T. |
| 248 | 98.1 | N.T. |
| 249 | 62.5 | N.T. |
| 250 | 95.2 | N.T. |
| 251 | 69.4 | N.T. |
| 252 | 70.4 | N.T. |
| 253 | N.T. | N.T. |
| 254 | N.T. | N.T. |
| 255 | N.T. | N.T. |
| 256 | N.T. | N.T. |
| 257 | N.T. | N.T. |
| 258 | N.T. | N.T. |
| 259 | N.T. | N.T. |
| 260 | N.T. | N.T. |
| 261 | N.T. | N.T. |
| 262 | N.T. | N.T. |
| 263 | N.T. | N.T. |

N.T. = not tested

___ - ___, means the compound was tested two or more times and the extreme values reported (for example, 23.7 - 26.6)

I.P. = Intraperitoneal administration

P.O. = oral administration

\+ = 80 mg/kg

* Tested, Data inconclusive (due to failure of untreated controls)

Toxic = Sheep died within 24 hr following treatment and were not examined for worms.

TABLE IIA

| Anthelmintic Activity (Adult Worms) or Compound 11 in Lambs When Administered Orally at 100 mg/kg (Experiment 1) | |
|---|---|
| Parasite | Percentage Clearance |
| Haemonchus contortus | 0 |
| Trichuris ovis | 14.5 |
| Oesophagostomum venulosum | 9.0 |
| Trichostrongylus axei | 52.7 |
| Trichostrongylus colubriformis | 0 |
| Trichostrongylus spp. | 47.9 |
| Nematodirus spathiger | 0 |
| Thysanosoma actinioides | 54.5 |

TABLE IIB

| Anthelmintic Activity (Adult Worms) of Compound #11 in Sheep When Administered Orally at 100/mg/kg (Experiment 2) | |
|---|---|
| Parasite | Percentage Clearance |
| Haemonchus | 0 |
| Ostertagia | 0 |
| Trichostrongylus axei | 0 |
| Trichostrongylus colubriformis | 42.0 |
| Cooperia | 0 |
| Nematodirus | 7.7 |
| Strongyloides | 33.9 |
| Trichuris | 53.4 |
| Oesphagostomum | 33.3 |

FORMULAE

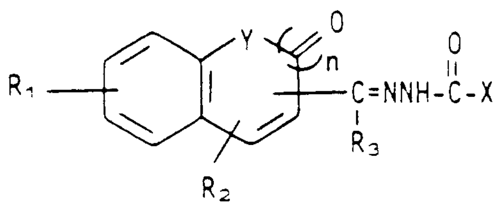

I

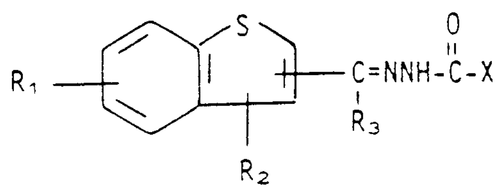

IA

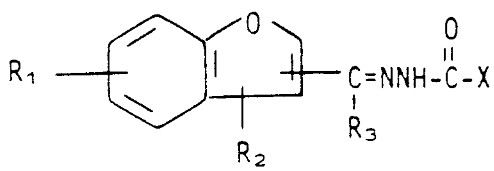

IB

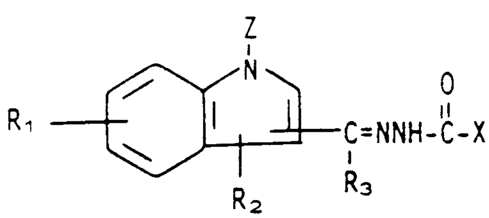

IC

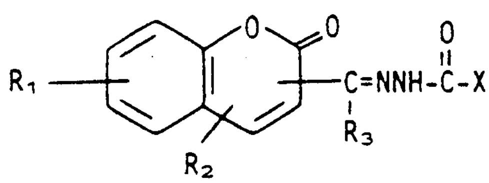

ID

CHART A

Scheme A:

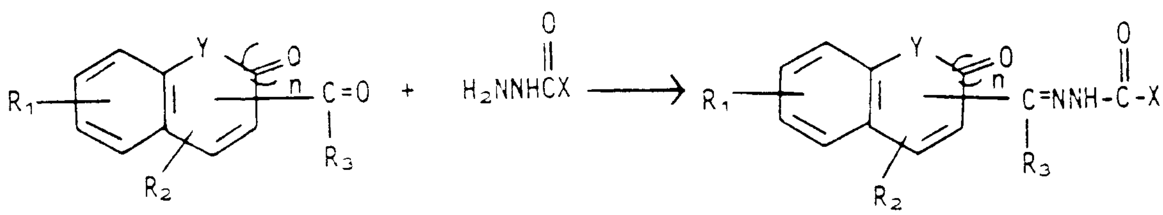

II III I

Scheme B:

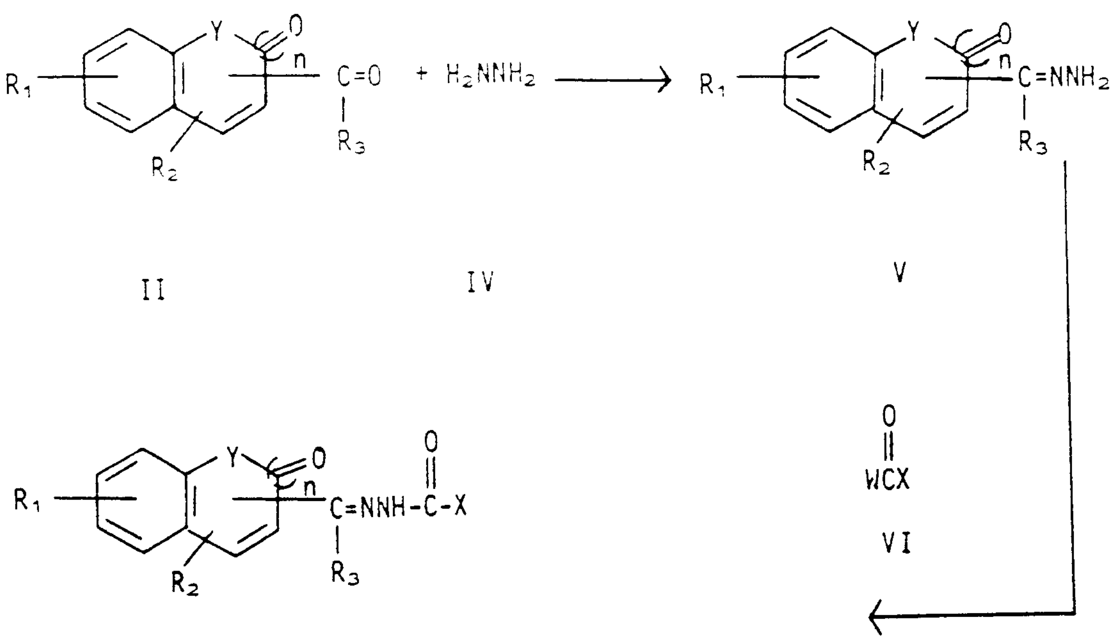

II IV V

VI

I

W is a halogen atom or other active group, for example, an anhydride

## Claims

### Claims for the following Contracting States : BE, CH, DE, FR, GB, IT, LU, NL, SE

**1.** A compound, for use in killing parasitic worms in humans and valuable warm-blooded domestic animals, which is an acylhydrazone, or hydrate or pharmaceutically-acceptable salt thereof, of the formula

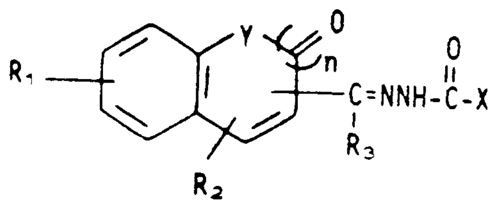

I

wherein X is (a) hydrogen; (b) $C_1$-$C_{10}$ alkyl; (c) $C_2$-$C_6$ alkenyl; (d) $C_2$-$C_6$ alkynyl; (e) cyclo($C_3$-$C_{10}$)alkyl optionally substituted with one, 2 or 3 $C_1$-$C_4$ alkyl, $C_2$-$C_4$ alkenyl, $C_1$-$C_4$ alkoxy, trifluoromethyl, or halo; (f) pyrrolidinyl; (g) piperidinyl; (h) 1-methylpyrrolidinyl; (i) 1-methylpiperidinyl; (j) $C_2$-$C_6$ alkyoxyalkyl; (k) cyclo($C_3$-$C_{10}$)alkyl($C_1$-$C_4$)alkyl; (1) phenyl($C_1$-$C_4$)alkyl optionally substituted with one, 2 or 3 halo or $C_1$-$C_4$ alkoxy, or one or 2 trifluoromethyl; (m) phenoxy($C_1$-$C_4$)alkyl optionally substituted with one, 2 or 3 halo, or one or 2 trifluoromethyl; (n) perhalo($C_1$-$C_7$)-alkyl; (o) $C_1$-$C_6$ alkoxy; (p) diphenylmethoxy; (q) cyclo($C_3$-$C_6$)alkyloxy optionally substituted with one or two $C_1$-$C_3$ alkyl; (r) phenoxy optionally substituted with one, 2 or 3 halo, or one or 2 trifluormethyl; (s) benzyloxy optionally substituted with one, 2 or 3 halo or $C_1$-$C_4$ alkoxy, or one or 2 trifluoromethyl; (t) heteroaromatic optionally substituted with one, 2 or 3 halo, or one or 2 trifluoromethyl; (u) phenyl substituted with one, 2 or 3 halo, or one or 2 trifluoromethyl, amino, alkyl($C_1$-$C_3$)amino, dialkyl($C_1$-$C_3$)amino, $C_1$-$C_5$ alkyl or $C_1$-$C_3$ alkoxy; (v) N-morpholinyl($C_1$-$C_4$)-alkyl; (w) N-piperidinyl($C_1$-$C_4$)alkyl; (x) N-pyrrolidinyl($C_1$-$C_4$)alkyl; (y) bridged polycyclic hydrocarbon substitutents of six to 10 nuclear carbons, optionally substituted with one, 2 or 3 ($C_1$-$C_3$) alkyl groups; or (z) naphthyl($C_1$-$C_3$)alkyl optionally substituted with one, 2 or 3 $C_1$-$C_4$ alkyl, $C_1$-$C_4$ alkoxy, halo, or trifluoromethyl;

wherein Y is an oxygen atom, a sulfur atom or a N-Z group;

wherein Z is hydrogen; $C_1$-$C_4$ alkyl; acetyl; benzoyl; phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; or phenyl($C_1$-$C_4$)alkyl optionally substituted with one, 2 or 3 $C_1$-$C_4$ alkyl, $C_1$-$C_4$ alkoxy, halo, or trifluoromethyl;

wherein $R_1$ is hydrogen; hydroxy; $C_1$-$C_4$ alkyl; $C_1$-$C_3$ alkoxy; $C_1$-$C_3$ alkylthio; halo; nitro; benzyloxy; or trifluoromethyl;

wherein $R_2$ is hydrogen; hydroxy; $C_1$-$C_4$ alkyl; $C_1$-$C_3$ alkoxy; $C_1$-$C_3$ alkylthio; halo; or trifluoromethyl;

wherein $R_3$ is hydrogen; $C_1$-$C_4$ alkyl;
cyclo($C_3$-$C_6$)alkyl optionally substituted with one, 2 or 3 $C_1$-$C_3$ alkyl; phenyl optionally substituted with one, 2 or 3 $C_1$-$C_4$ alkyl, halo, trifluoromethyl or $C_1$-$C_3$ alkoxy; phenyl($C_1$-$C_3$)alkyl optionally substituted with one, 2 or 3 $C_1$-$C_4$ alkyl, halo, trifluoromethyl or $C_1$-$C_3$ alkoxy; or 1,3-dioxacyclohexan-5-yl; and

n is 0 or 1;

with the provisos that

(i) n is 0 when Y is a sulfur atom or a N-Z group;

(ii) when X is n-propyl, $R_3$ is methyl, $R_1$ and $R_2$ are hydrogen, the compound is other than an indol-3-yl acylhydrazone;

(iii) when X is cyclopropyl, $R_2$ is 2-methyl, $R_1$ and $R_3$ are hydrogen, the compound is other than a indol-3-yl acylhydrazone;

(iv) when X is cyclohexylmethyl, $R_1$ and $R_2$ are hydrogen, $R_3$ is ethyl, the compound is other than 1-benzylindol-3-yl acylhydrazone;

(v) when X is t-butoxy, the compound is a 2-benzofuranyl acylhydrazone, and $R_1$ and $R_2$ are hydrogen, $R_3$ is other than methyl;

(vi) when X is ethoxy, $R_1$, $R_2$ and $R_3$ are hydrogen, the compound is other than a 1-acetylindol-3-yl acylhydrazone;

(vii) when X is ethoxy, $R_1$ and $R_2$ are hydrogen, $R_3$ is ethyl, the compound is other than a 1-benzylindol-3-yl acylhydrazone;

(viii) when X is ethoxy, $R_1$ and $R_2$ are hydrogen, $R_3$ is methyl, the compound is other than a 3-benzothiofuran acylhydrazone;

(ix) when X is phenoxy, $R_1$ and $R_2$ are hydrogen and $R_3$ is methyl, the compound is other than a 2-benzofuranyl acylhydrazone;

(x) when X is benzyloxy and $R_1$, $R_2$ and $R_3$ are hydrogen, the compound is other than a indol-3-yl acylhydrazone;

(xi) when X is 4-ethyoxyphenyl or 4-chlorophenyl, $R_1$ and $R_2$ are hydrogen, $R_3$ is methyl, the compound is other than a 3-benzothienyl acylhydrazone;
(xii) when X is 4-chlorophenyl, $R_1$ and $R_2$ are hydrogen, $R_3$ is ethyl, the compound is other than a 1-benzylindol-3-yl acylhydrazone;
(xiii) when X is 4-methylphenyl, 3,4-dimethoxyphenyl or 3-methoxyphenyl and $R_1$, $R_2$ and $R_3$ are hydrogen, the compound is other than a indol-3-yl acylhydrazone;
(iv) when X is 4-ethoxyphenyl, $R_1$, $R_2$ and $R_3$ are hydrogen, the compound is other than a 1-acetylindol-3-yl acylhydrazone; and
(xv) when $R_1$ is benzyloxy, X is other than alkyl.

**2.** A compound for use according to claim 1, wherein n is 0.

**3.** A compound for use according to claim 2, wherein Y is NH.

**4.** A compound for use according to claim 3, which is a 1H-indol-3-yl acylhydrazone.

**5.** A compound for use according to any preceding claim, wherein X is cyclobutyl, t-butoxy, ethoxy, ethyl or 3-pyridinyl.

**6.** A compound for use according to any preceding claim, wherein $R_1$ and $R_2$ are independently selected from hydrogen, methyl and bromo.

**7.** A compound for use according to any preceding claim, wherein $R_3$ is hydrogen.

**8.** A compound for use according to claim 1, which is
1,1-dimethylethyl [(1H-indol-3-yl)methylene]carbazate (Cpd 4);
ethyl [(1H-indol-3-yl)methylene]carbazate (Cpd 12);
nicotinic acid [(1H-indol-3-yl)methylene]hydrazide (Cpd 13);
ethyl [(5-bromo-1H-indol-3-yl)methylene]carbazate (Cpd 52);
cyclobutanecarboxylic acid [(1H-indol-3-yl)methylene]hydrazide (Cpd 66);
ethyl [(2-methyl-1H-indol-3-yl)methylene]carbazate (Cpd 67);
butanoic acid [(2-methyl-1H-indol-3-yl)methylene]hydrazide (Cpd 69);
propanoic acid ((2-methyl-1H-indol-3-yl)methylene]hydrazide (Cpd 70);
nicotinic acid [(2-methyl-1H-indol-3-yl)methylene]hydrazide (Cpd 96);
1,1-dimethylethyl [(2-methyl-1H-indol-3-yl)methylene]hydrazide (Cpd 97);
propanoic acid [(1-acetylindol-3-yl)methylene]hydrazide (Cpd 145); or
phenylacetic acid [(2-methyl-1H-indol-3-yl)methylene]hydrazide (Cpd 167).

**9.** A compound for use according to claim 1, which is
acetic acid [(1-phenylmethylindol-3-yl)propylidene]hydrazide (Cpd 64).

**10.** A compound for use according to claim 1, which is
ethyl [(1H-indol-3-yl)ethylidene]carbazate (Cpd 79).

**11.** A compound for use according to claim 1, which is
formic acid [1-(3-benzofuranyl)ethylidene]hydrazide (Cpd 81).

**12.** A compound for use according to claim 1, which is
butanoic acid [1-(3-benzothienyl)ethylidene]hydrazide (Cpd 118);

**13.** A compound for use according to claim 1, which is
ethyl [1-(2-oxo-2H-1-benzopyran-3-yl)ethylidene]carbazate (Cpd 248); or
butanoic acid [1-(2-oxo-2H-1-benzopyran-3-yl)ethylidene]hydrazide (Cpd 250).

**14.** An acylhydrazone, or hydrate or pharmaceutically-acceptable salt thereof, of the formula defined in any preceding claim, other than
acetic acid [(1H-indol-3-yl)methylene]hydrazide, (CA RN 1016-49-5);
4-aminobenzoic acid [(1H-indol-3-yl)methylene]hydrazide, (CA RN 10245-42-8);

4-methoxybenzoic acid [(1-methylindol-2-yl)methylene]hydrazide, (CA RN 15565-86-3);
4-morpholineacetic acid (2-benzofuranylmethylene)hydrazide, (CA RN 72627-58-8);
(2-chlorophenoxy)acetic acid (1H-indol-3-yl-methylene)hydrazide, (CA RN 81111-25-3);
(4-chlorophenoxy)acetic acid (1H-indol-3-yl-methylene)hydrazide, (CA RN 81111-26-4);
(2-chlorophenoxy)acetic acid [(2-methoxy-1H-indol-3-yl)methylene]hydrazide, (CA RN 81122-74-9);
(4-chlorophenoxy)acetic acid [(2-methoxy-1H-indol-3-yl)methylene]hydrazide (CA RN 81122-75-0);
methyl (1H-indol-3-ylmethylene)carbazate (CA RN 88692-99-3);
phenylmethyl [1-(1H-indol-3-yl)propylidene]carbazate (CA RN 32947-16-3);
1-piperidineacetic acid [(2-methyl-3-benzofuranyl)methylene]hydrazide (CA RN 35806-44-1);
1-pyrrolidineacetic acid (2-benzofuranylmethylene)hydrazide (CA RN 35806-70-3);
1-piperidineacetic acid (2-benzofuranylmethylene)hydrazide (CA RN 35806-85-0);
1-pyrrolidineacetic acid [(2-methyl-3-benzofuranyl)methylene]hydrazide (CA RN 35802-00-9);
formic acid [(1H-indol-2-yl)methylene]hydrazide (CA RN 50335-88-1);
ethyl (1H-indol-2-ylmethylene)carbazate (CA RN 50335-89-2);
ethyl [1-(2-benzofuranyl)ethylidene]carbazate (CA RN 56968-94-6);
ethyl [1-(5-chloro-2-benzofuranyl)ethylidene]carbazate (CA RN 56968-95-7);
1,1-dimethylethyl (1H-indol-3-ylmethylene)carbazate (CA RN 57699-52-2);
4-aminobenzoic acid [(1H-indol-3-yl)methylene]hydrazide (CA RN 15641-21-1);
butyric acid [(1H-indol-3-yl)methylene]hydrazide (CA RN 15641-24-4);
propionic acid [(1H-indol-3-yl)methylene]hydrazide (CA RN 15641-25-5);
ethyl [(1H-indol-3-yl)methylene]carbazate (CA RN 15641-27-7);
formic acid [(1-methylindol-2-yl)methylene]hydrazide (CA RN 15641-28-8);
acetic acid [(1-methylindol-2-yl)methylene]hydrazide (CA RN 15641-29-9);
propanoic acid [(1-methylindol-2-yl)methylene]hydrazide (CA RN 15641-30-2);
butanoic acid [(1-methylindol-2-yl)methylene]hydrazide (CA RN 15641-31-3);
2-methylpropanoic acid [(1-methylindol-2-yl)methylene]hydrazide (CA RN 15641-32-4);
phenylacetic acid [(1-methylindol-2-yl)methylene]hydrazide (CA RN 15641-34-6);
4-chlorobenzoic acid [(1-methylindol-2-yl)methylene]hydrazide (CA RN 15641-36-8);
1-naphthaleneacetic acid [(1-methylindol-2-yl)methylene]hydrazide(CA RN 15641-38-0);
ethyl [(1-methylindol-2-yl)methylene]carbazate (CA RN 15565-88-5);
4-chlorobenzoic acid [(1H-indol-3-yl)methylene]hydrazide (CA RN 15641-01-7);
2-chlorobenzoic acid [(1H-indol-3-yl)methylene]hydrazide (CA RN 15641-03-9);
3-chlorobenzoic acid [(1H-indol-3-yl)methylene]hydrazide (CA RN 15641-04-0);
2,4-dichlorobenzoic acid [(1H-indol-3-yl)methylene]hydrazide (CA RN 15641-05-1);
4-bromobenzoic acid [(1H-indol-3-yl)methylene]hydrazide (CA RN 15641-06-2);
2-methylpropanoic acid [(1H-indol-3-yl)methylene]hydrazide (CA RN 15641-12-0);
4-methoxybenzoic acid [(1H-indol-3-yl)methylene]hydrazide (CA RN 15641-15-3);
phenylacetic acid [(1H-indol-3-yl)methylene]hydrazide (CA RN 15641-17-5);
1-naphthaleneacetic acid [(1H-indol-3-yl)methylene]hydrazide (CA RN 15641-18-6);
2-furoic acid [(1H-indol-3-yl)methylene]hydrazide (CA RN 15641-19-7);
2-naphthaleneacetic acid [(1H-indol-3-yl)methylene]hydrazide (CA RN 15718-86-2);
ethyl [1-(1H-indol-3-yl)ethylidene]carbazate (CA RN 32927-03-0);
phenylmethyl 3-[(1H-indol-3-yl)methylene]carbazate (CA RN 32927-05-2);
phenylmethyl [1-(1H-indol-3-yl)ethylidene]carbazate (CA RN 32927-06-3); or
ethyl [(1-methylindol-3-yl)methylene]carbazate (CA RN 32927-89-2).

**15.** Use of a compound according to any preceding claim, for the manufacture of a medicament for use in killing parasitic worms in human and valuable warm-blooded domestic animals.

**Claims for the following Contracting State : AT**

**1.** A process for preparing an acylhydroazone, or hydrate or pharmaceutically-acceptable salt thereof, of the formula

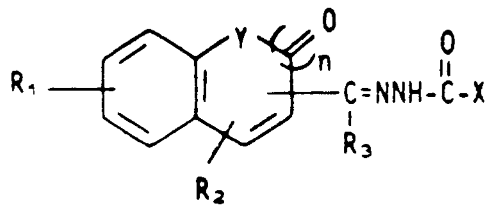

I

which comprises either (1) reacting corresponding compounds of formulae II and III

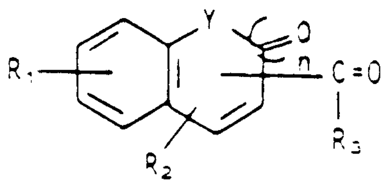

II

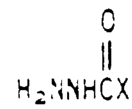

III

or (2) reacting the compound of formula II with hydrazine and the resulting hydrazone with a corresponding compound of the formula WCOX;
wherein W is a halogen atom, an anhydride or another reactive group;
wherein X is (a) hydrogen; (b) $C_1$-$C_{10}$ alkyl; (c) $C_2$-$C_6$ alkenyl; (d) $C_2$-$C_6$ alkynyl; (e) cyclo($C_3$-$C_{10}$)alkyl optionally substituted with one, 2 or 3 $C_1$-$C_4$ alkyl, $C_2$-$C_4$ alkenyl, $C_1$-$C_4$ alkoxy, trifluoromethyl, or halo; (f) pyrrolidinyl; (g) piperidinyl; (h) 1-methylpyrrolidinyl; (i) 1-methylpiperidinyl; (j) $C_2$-$C_6$ alkyoxyalkyl; (k) cyclo($C_3$-$C_{10}$)alkyl($C_1$-$C_4$)alkyl; (1) phenyl($C_1$-$C_4$)alkyl optionally substituted with one, 2 or 3 halo or $C_1$-$C_4$ alkoxy, or one or 2 trifluoromethyl; (m) phenoxy($C_1$-$C_4$)alkyl optionally substituted with one, 2 or 3 halo, or one or 2 trifluoromethyl; (n) perhalo($C_1$-$C_7$)-alkyl; (o) $C_1$-$C_6$ alkoxy; (p) diphenylmethoxy; (q) cyclo($C_3$-$C_6$)alkyloxy optionally substituted with one or two $C_1$-$C_3$ alkyl; (r) phenoxy optionally substituted with one, 2 or 3 halo, or one or 2 trifluormethyl; (s) benzyloxy optionally substituted with one, 2 or 3 halo or $C_1$-$C_4$ alkoxy, or one or 2 trifluoromethyl; (t) heteroaromatic optionally substituted with one, 2 or 3 halo, or one or 2 trifluoromethyl; (u) phenyl substituted with one, 2 or 3 halo, or one or 2 trifluoromethyl, amino, alkyl($C_1$-$C_3$)amino, dialkyl($C_1$-$C_3$)amino, $C_1$-$C_5$ alkyl or $C_1$-$C_3$ alkoxy; (v) N-morpholinyl ($C_1$-$C_4$)-alkyl; (w) N-piperidinyl($C_1$-$C_4$)alkyl; (x) N-pyrrolidinyl($C_1$-$C_4$)alkyl; (y) bridged polycyclic hydrocarbon substitutents of six to 10 nuclear carbons, optionally substituted with one, 2 or 3 ($C_1$-$C_3$) alkyl groups; or (z) naphthyl($C_1$-$C_3$)alkyl optionally substituted with one, 2 or 3 $C_1$-$C_4$ alkyl, $C_1$-$C_4$ alkoxy, halo, or trifluoromethyl;

wherein Y is an oxygen atom, a sulfur atom or a N-Z group;

wherein Z is hydrogen; $C_1$-$C_4$ alkyl; acetyl; benzoyl; phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; or phenyl($C_1$-$C_4$)alkyl optionally substituted with one, 2 or 3 $C_1$-$C_4$ alkyl, $C_1$-$C_4$ alkoxy, halo, or trifluoromethyl;

wherein $R_1$ is hydrogen; hydroxy; $C_1$-$C_4$ alkyl; $C_1$-$C_3$ alkoxy; $C_1$-$C_3$ alkylthio; halo; nitro; benzyloxy; or trifluoromethyl;

wherein $R_2$ is hydrogen; hydroxy; $C_1$-$C_4$ alkyl; $C_1$-$C_3$ alkoxy; $C_1$-$C_3$ alkylthio; halo; or trifluoromethyl;

wherein $R_3$ is hydrogen; $C_1$-$C_4$ alkyl; cyclo($C_3$-$C_6$)alkyl optionally substituted with one, 2 or 3 $C_1$-$C_3$ alkyl; phenyl optionally substituted with one, 2 or 3 $C_1$-$C_4$ alkyl, halo, trifluoromethyl or $C_1$-$C_3$ alkoxy; phenyl($C_1$-$C_3$)alkyl optionally substituted with one, 2 or 3 $C_1$-$C_4$ alkyl, halo, trifluoromethyl or $C_1$-$C_3$ alkoxy; or 1,3-dioxacyclohexan-5-yl; and

n is 0 or 1;

with the provisos that

(i) n is 0 when Y is a sulfur atom or a N-Z group;

(ii) when X is n-propyl, $R_3$ is methyl, $R_1$ and $R_2$ are hydrogen, the compound is other than an indol-3-yl acylhydrazone;

(iii) when X is cyclopropyl, $R_2$ is 2-methyl, $R_1$ and $R_3$ are hydrogen, the compound is other than a

indol-3-yl acylhydrazone;
(iv) when X is cyclohexylmethyl, $R_1$ and $R_2$ are hydrogen, $R_3$ is ethyl, the compound is other than 1-benzylindol-3-yl acylhydrazone;
(v) when X is t-butoxy, the compound is a 2-benzofuranyl acylhydrazone, and $R_1$ and $R_2$ are hydrogen, $R_3$ is other than methyl;
(vi) when X is ethoxy, $R_1$, $R_2$ and $R_3$ are hydrogen, the compound is other than a 1-acetylindol-3-yl acylhydrazone;
(vii) when X is ethoxy, $R_1$ and $R_2$ are hydrogen, $R_3$ is ethyl, the compound is other than a 1-benzylindol-3-yl acylhydrazone;
(viii) when X is ethoxy, $R_1$ and $R_2$ are hydrogen, $R_3$ is methyl, the compound is other than a 3-benzothiofuran acylhydrazone;
(ix) when X is phenoxy, $R_1$ and $R_2$ are hydrogen and $R_3$ is methyl, the compound is other than a 2-benzofuranyl acylhydrazone;
(x) when X is benzyloxy and $R_1$, $R_2$ and $R_3$ are hydrogen, the compound is other than a indol-3-yl acylhydrazone;
(xi) when X is 4-ethyoxyphenyl or 4-chlorophenyl, $R_1$ and $R_2$ are hydrogen, $R_3$ is methyl, the compound is other than a 3-benzothienyl acylhydrazone;
(xii) when X is 4-chlorophenyl, $R_1$ and $R_2$ are hydrogen, $R_3$ is ethyl, the compound is other than a 1-benzylindol-3-yl acylhydrazone;
(xiii) when X is 4-methylphenyl, 3,4-dimethoxyphenyl or 3-methoxyphenyl and $R_1$, $R_2$ and $R_3$ are hydrogen, the compound is other than a indol-3-yl acylhydrazone;
(xiv) when X is 4-ethoxyphenyl, $R_1$, $R_2$ and $R_3$ are hydrogen, the compound is other than a 1-acetylindol-3-yl acylhydrazone;
(xv) when $R_1$ is benzyloxy, X is other than alkyl; and
(xvi) the acylhydrazone, or hydrate or pharmaceutically-acceptable salt thereof, is other than
acetic acid [(1H-indol-3-yl)methylene]hydrazide, (CA RN 1016-49-5);
4-aminobenzoic acid [(1H-indol-3-yl)methylene]hydrazide, (CA RN 10245-42-8);
4-methoxybenzoic acid [(1-methylindol-2-yl)methylene]hydrazide, (CA RN 15565-86-3);
4-morpholineacetic acid (2-benzofuranylmethylene)hydrazide, (CA RN 72627-58-8);
(2-chlorophenoxy)acetic acid (1H-indol-3-yl-methylene)hydrazide, (CA RN 81111-25-3);
(4-chlorophenoxy)acetic acid (1H-indol-3-yl-methylene)hydrazide, (CA RN 81111-26-4);
(2-chlorophenoxy)acetic acid [(2-methoxy-1H-indol-3-yl)methylene]hydrazide, (CA RN 81122-74-9);
(4-chlorophenoxy)acetic acid [(2-methoxy-1H-indol-3-yl)methylene]hydrazide (CA RN 81122-75-0);
methyl (1H-indol-3-ylmethylene)carbazate (CA RN 88692-99-3);
phenylmethyl [1-(1H-indol-3-yl)propylidene carbazate (CA RN 32947-16-3);
1-piperidineacetic acid [(2-methyl-3-benzofuranyl)methylene]hydrazide (CA RN 35806-44-1);
1-pyrrolidineacetic acid (2-benzofuranylmethylene)hydrazide (CA RN 35806-70-3);
1-piperidineacetic acid (2-benzofuranylmethylene)hydrazide (CA RN 35806-85-0);
1-pyrrolidineacetic acid [(2-methyl-3-benzofuranyl)methylene]hydrazide (CA RN 35802-00-9);
formic acid [(1H-indol-2-yl)methylene]hydrazide (CA RN 50335-88-1);
ethyl (1H-indol-2-ylmethylene)carbazate (CA RN 50335-89-2);
ethyl [1-(2-benzofuranyl)ethylidene]carbazate (CA RN 56968-94-6);
ethyl [1-(5-chloro-2-benzofuranyl)ethylidene]carbazate (CA RN 56968-95-7);
1,1-dimethylethyl (1H-indol-3-ylmethylene)carbazate (CA RN 57699-52-2);
4-aminobenzoic acid [(1H-indol-3-yl)methylene]hydrazide (CA RN 15641-21-1);
butyric acid [(1H-indol-3-yl)methylene]hydrazide (CA RN 15641-24-4);
propionic acid [(1H-indol-3-yl)methylene]hydrazide (CA RN 15641-25-5);
ethyl [(1H-indol-3-yl)methylene]carbazate (CA RN 15641-27-7);
formic acid [(1-methylindol-2-yl)methylene]hydrazide (CA RN 15641-28-8);
acetic acid [(1-methylindol-2-yl)methylene]hydrazide (CA RN 15641-29-9);
propanoic acid [(1-methylindol-2-yl)methylene]hydrazide (CA RN 15641-30-2);
butanoic acid [(1-methylindol-2-yl)methylene]hydrazide (CA RN 15641-31-3);
2-methylpropanoic acid [(1-methylindol-2-yl)methylene]hydrazide (CA AN 15641-32-4);
phenylacetic acid [(1-methylindol-2-yl)methylene]hydrazide (CA RN 15641-34-6);
4-chlorobenzoic acid [(1-methylindol-2-yl)methylene]hydrazide (CA RN 15641-36-8);
1-naphthaleneacetic acid [(1-methylindol-2-yl)methylene]hydrazide(CA RN 15641-38-0);

ethyl [(1-methylindol-2-yl)methylene]carbazate (CA RN 15565-88-5);
4-chlorobenzoic acid [(1H-indol-3-yl)methylene]hydrazide (CA RN 15641-01-7);
2-chlorobenzoic acid [(1H-indol-3-yl)methylene]hydrazide (CA RN 15641-03-9);
3-chlorobenzoic acid [(1H-indol-3-yl)methylene]hydrazide (CA RN 15641-04-0);
2,4-dichlorobenzoic acid [(1H-indol-3-yl)methylene]hydrazide (CA RN 15641-05-1);
4-bromobenzoic acid [(1H-indol-3-yl)methylene]hydrazide (CA RN 15641-06-2);
2-methylpropanoic acid [(1H-indol-3-yl)methylene]hydrazide (CA RN 15641-12-0);
4-methoxybenzoic acid [(1H-indol-3-yl)methylene]hydrazide (CA RN 15641-15-3);
phenylacetic acid [(1H-indol-3-yl)methylene]hydrazide (CA RN 15641-17-5);
1-naphthaleneacetic acid [(1H-indol-3-yl)methylene]hydrazide (CA RN 15641-18-6);
2-furoic acid [(1H-indol-3-yl)methylene]hydrazide (CA RN 15641-19-7);
2-naphthaleneacetic acid [(1H-indol-3-yl)methylene]hydrazide (CA RN 15718-86-2):
ethyl [1-(1H-indol-3-yl)ethylidene]carbazate (CA RN 32927-03-0);
phenylmethyl 3-[(1H-indol-3-yl)methylene]carbazate (CA RN 32927-05-2);
phenylmethyl [1-(1H-indol-3-yl)ethylidene]carbazate (CA RN 32927-06-3); or
ethyl [(1-methylindol-3-yl)methylene]carbazate (CA RN 32927-89-2).

**2.** A process according to claim 1, wherein n is 0.

**3.** A process according to claim 2, wherein Y is NH.

**4.** A process according to claim 3, wherein the acylhydrazone is a 1H-indol-3-yl acylhydrazone.

**5.** A process according to any preceding claim, wherein X is cyclobutyl, t-butoxy, ethoxy, ethyl or 3-pyridinyl.

**6.** A process according to any preceding claim, wherein $R_1$ and $R_2$ are independently selected from hydrogen, methyl and bromo.

**7.** A process according to any preceding claim, wherein $R_3$ is hydrogen.

**8.** A process according to claim 1, wherein the acylhydrazone is
1,1-dimethylethyl [(1H-indol-3-yl)methylene]carbazate (Cpd 4);
nicotinic acid [(1H-indol-3-yl)methylene]hydrazide (Cpd 13);
ethyl [(5-bromo-1H-indol-3-yl)methylene]carbazate (Cpd 52);
cyclobutanecarboxylic acid [(1H-indol-3-yl)methylene]hydrazide (Cpd 66);
ethyl [(2-methyl-1H-indol-3-yl)methylene]carbazate (Cpd 67);
butanoic acid [(2-methyl-1H-indol-3-yl)methylene]hydrazide (Cpd 69);
propanoic acid [(2-methyl-1H-indol-3-yl)methylene]hydrazide (Cpd 70);
nicotinic acid [(2-methyl-1H-indol-3-yl)methylene]hydrazide (Cpd 96);
1,1-dimethylethyl [(2-methyl-1H-indol-3-yl)methylene]hydrazide (Cpd 97);
propanoic acid [(1-acetylindol-3-yl)methylene]hydrazide (Cpd 145); or
phenylacetic acid [(2-methyl-1H-indol-3-yl)methylene]hydrazide (Cpd 167).

**9.** A process according to claim 1, wherein the acylhydrazone is
acetic acid [(1-phenylmethylindol-3-yl)propylidene]hydrazide (Cpd 64).

**10.** A process according to claim 1, wherein the acylhydrazone is
formic acid [1-(3-benzofuranyl)ethylidene]hydrazide (Cpd 81).

**11.** A process according to claim 1, wherein the acylhydrazone is
butanoic acid [1-(3-benzothienyl)ethylidene]hydrazide (Cpd 118);

**12.** A process according to claim 1, wherein the acylhydrazone is
ethyl [1-(2-oxo-2H-1-benzopyran-3-yl)ethylidene]carbazate (Cpd 248); or
butanoic acid [1-(2-oxo-2H-1-benzopyran-3-yl)ethylidene]hydrazide (Cpd 250).

**Revendications**

**Revendications pour les Etats contractants suivants : BE, CH, DE, FR, GB, IT, LU, NL, SE,**

1. Composé destiné à être utilisé pour détruire des vers parasites chez l'homme et les animaux à sang chaud domestiques de valeur, qui est une acylhydrazone ou un hydrate ou un sel pharmaceutiquement acceptable de ce composé, de formule :

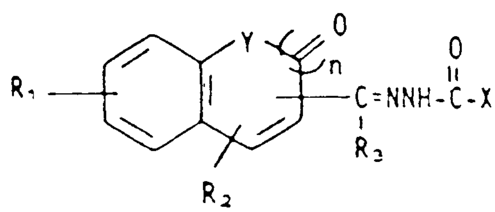

I

dans laquelle X représente (a) l'hydrogène ; (b) un groupe alkyle en $C_1$ à $C_{10}$ ; (c) un groupe alcényle en $C_2$ à $C_6$ ; (d) un groupe alcynyle en $C_2$ à $C_6$ ; (e) un groupe cycloalkyle en $C_3$ à $C_{10}$ facultativement substitué avec 1, 2 ou 3 radicaux alkyle en $C_1$ à $C_4$, alcényle en $C_2$ à $C_4$, alkoxy en $C_1$ à $C_4$, trifluorométhyle ou halogéno ; (f) un groupe pyrrolidinyle ; (g) un groupe pipéridinyle ; (h) un groupe 1-méthylpyrrolidinyle ; (i) un groupe 1-méthylpipéridinyle ; (j) un groupe alkoxyalkyle en $C_2$ à $C_6$ ; (k) un groupe (cycloalkyle en $C_3$ à $C_{10}$)-(alkyle en $C_1$ à $C_4$) ; (1) un groupe phényl-(alkyle en $C_1$ à $C_4$) facultativement substitué avec 1, 2 ou 3 radicaux halogéno ou alkoxy en $C_1$ à $C_4$, ou bien 1 ou 2 radicaux trifluorométhyle ; (m) un groupe phénoxy-(alkyle en $C_1$ à $C_4$) facultativement substitué avec 1, 2 ou 3 radicaux halogéno, ou avec 1 ou 2 radicaux trifluorométhyle ; (n) un groupe perhalogénalkyle en $C_1$ à $C_7$ ; (o) un groupe alkoxy en $C_1$ à $C_6$ ; (p) un groupe diphénylméthoxy ; (q) un groupe cycloalkyloxy en $C_3$ à $C_6$ facultativement substitué avec un ou deux radicaux alkyle en $C_1$ à $C_3$ ; (r) un groupe phénoxy facultativement substitué avec 1, 2 ou 3 radicaux halogéno,ou avec 1 ou 2 radicaux trifluorométhyle ; (s) un groupe benzyloxy facultativement substitué avec 1, 2 ou 3 radicaux halogéno ou alkoxy en $C_2$ à $C_4$ ou avec 1 ou 2 radicaux trifluorométhyle ; (t) un groupe hétéro-aromatique facultativement substitué avec 1, 2 ou 3 radicaux halogéno, ou avec 1 ou 2 radicaux trifluorométhyle ; (u) un groupe phényle substitué avec 1, 2 ou 3 radicaux halogéno, ou avec 1 ou 2 radicaux trifluorométhyle, amino, alkylamino en $C_1$ à $C_3$, di(alkyle en $C_1$ à $C_3$)amino, alkyle en $C_1$ à $C_5$ ou alkoxy en $C_1$ à $C_3$ ; (v) un groupe N-morpholinyl-(alkyle en $C_1$ à $C_4$) ; (w) un groupe N-pipéridinyl-(alkyle en $C_1$ à $C_4$) ; (x) un groupe N-pyrrolidinyl-(alkyle en $C_1$ à $C_4$) ; (y) des substituants hydrocarbonés polycycliques pontés ayant 6 à 10 atomes cycliques, facultativement substitués avec 1, 2 ou 3 groupes alkyle en $C_1$ à $C_3$ ; ou (z) un groupe naphtyl(alkyle en $C_1$ à $C_3$) facultativement substitué avec 1, 2 ou 3 radicaux alkyle en $C_1$ à $C_4$, alkoxy en $C_1$ à $C_4$, halogéno ou trifluorométhyle ;

Y est un atome d'oxygène, un atome de soufre ou un groupe N-Z ;

Z est de l'hydrogène ; un groupe alkyle en $C_1$ à $C_4$ ; acétyle ; benzoyle ; phényle facultativement substitué avec 1, 2 ou 3 radicaux alkyle en $C_1$ à $C_4$, alkoxy en $C_1$ à $C_3$, halogéno, trifluorométhyle, dialkylamino en $C_2$ à $C_6$, alkylthio en $C_1$ à $C_3$, nitro ou phénoxy facultativement substitué avec 1, 2 ou 3 radicaux alkyle en $C_1$ à $C_4$, alkoxy en $C_1$ à $C_3$, halogéno ou trifluorométhyle ; ou un groupe phényl-(alkyle en $C_1$ à $C_4$) facultativement substitué avec 1, 2 ou 3 radicaux alkyle en $C_1$ à $C_4$, alkoxy en $C_1$ à $C_4$, halogéno ou trifluorométhyle ;

$R_1$ est l'hydrogène ; un groupe hydroxy ; alkyle en $C_1$ à $C_4$ ; alkoxy en $C_1$ à $C_3$ ; alkylthio en $C_1$ à $C_3$ ; halogéno ; nitro ; benzyloxy ; ou trifluorométhyle ;

$R_2$ est l'hydrogène ; un groupe hydroxy ; alkyle en $C_1$ à $C_4$ ; alkoxy en $C_1$ à $C_3$ ; alkylthio en $C_1$ à $C_3$ ; halogéno : ou trifluorométhyle ;

$R_3$ est l'hydrogène ; un groupe alkyle en $C_1$ à $C_4$ ; un groupe cycloalkyle en $C_3$ à $C_6$ facultativement substitué avec 1, 2 ou 3 radicaux alkyle en $C_1$ à $C_3$ ; un groupe phényle facultativement substitué avec 1, 2 ou 3 radicaux alkyle en $C_1$ à $C_4$, halogéno, trifluorométhyle ou alkoxy en $C_1$ à $C_3$ ; phényl-(alkyle en $C_1$ à $C_3$) facultativement substitué avec 1, 2 ou 3 radicaux alkyle en $C_1$ à $C_4$, halogéno, trifluorométhyle ou alkoxy en $C_1$ à $C_3$ ; ou un groupe 1,3-dioxacyclohexane-5-yle ; et

$\underline{n}$ a la valeur 0 ou 1 ;

sous réserve que

(i) $\underline{n}$ soit égal à 0 lorsque Y est un atome de soufre ou un groupe N-Z ;

(ii) lorsque X est un groupe n-propyle, $R_3$ est un groupe méthyle, $R_1$ et $R_2$ représentent de l'hydrogène, le composé soit autre chose qu'une indole-3-ylacylhydrazone ;

(iii) lorsque X est un groupe cyclopropyle, $R_2$ est un groupe 2-méthyle, $R_1$ et $R_3$ sont de l'hydrogène, le composé soit autre chose qu'une indole-3-ylacylhydrazone ;
(iv) lorsque X est un groupe cyclohexylméthyle, $R_1$ et $R_2$ sont de l'hydrogène, $R_3$ est un groupe éthyle, le composé soit autre chose que la 1-benzylindole-3-yl-acylhydrazone ;
(v) lorsque X est un groupe tertio-butoxy, le composé soit une 2-benzofurannylacylhydrazone, et lorsque $R_1$ et $R_2$ sont de l'hydrogène, $R_3$ soit autre chose qu'un groupe méthyle ;
(vi) lorsque X est un groupe éthoxy, $R_1$, $R_2$ et $R_3$ sont de l'hydrogène, le composé soit autre chose qu'une 1-acétylindole-3-ylacylhydrazone ;
(vii) lorsque X est un groupe éthoxy, $R_1$ et $R_2$ sont de l'hydrogène, $R_3$ est un groupe éthyle, le composé soit autre chose qu'une 1-benzylindole-3-ylacylhydrazone ;
(viii) lorsque X est un groupe éthoxy, $R_1$ et $R_2$ sont de l'hydrogène, $R_3$ est un groupe méthyle, le composé soit autre chose qu'une 3-benzothiofuranne-acylhydrazone ;
(ix) lorsque X est un groupe phénoxy, $R_1$ et $R_2$ sont de l'hydrogène et $R_3$ est un groupe méthyle, le composé soit autre chose qu'une 2-benzofurannylacylhydrazone ;
(x) lorsque X est un groupe benzyloxy et $R_1$, $R_2$ et $R_3$ sont de l'hydrogène, le composé soit autre chose qu'une indole-3-ylacylhydrazone ;
(xi) lorsque X est un groupe 4-éthyloxyphényle ou 4-chlorophényle, $R_1$ et $R_2$ sont de l'hydrogène, $R_3$ est un groupe méthyle, le composé soit autre chose qu'une 3-benzothiénylacylhydrazone ;
(xii) lorsque X est un groupe 4-chlorophényle, $R_1$ et $R_2$ sont de l'hydrogène, $R_3$ est un groupe éthyle, le composé soit autre chose qu'une 1-benzylindole-3-ylacylhydrazone ;
(xiii) lorsque X est un groupe 4-méthylphényle, 3,4-diméthoxyphényle ou 3-méthoxyphényle et $R_1$, $R_2$ et $R_3$ sont de l'hydrogène, le composé soit autre chose qu'une indole-3-ylacylhydrazone ;
(xiv) lorsque X est un groupe 4-éthoxyphényle, $R_1$, $R_2$ et $R_3$ sont de l'hydrogène, le composé soit autre chose qu'une 1-acétylindole-3-ylacylhydrazone ; et
(xv) lorsque $R_1$ est un groupe benzyloxy, X soit autre chose qu'un groupe alkyle.

**2.** Composé destiné à être utilisé suivant la revendication 1, dans lequel $\underline{n}$ est égal à 0.

**3.** Composé destiné à être utilisé suivant la revendication 2, dans lequel Y est un groupe NH.

**4.** Composé destiné à être utilisé suivant la revendication 3, qui est une 1H-indole-3-ylacylhydrazone.

**5.** Composé destiné à être utilisé suivant l'une quelconque des revendications précédentes, dans lequel X est un groupe cyclobutyle, tertio-butoxy, éthoxy, éthyle ou 3-pyridinyle.

**6.** Composé destiné à être utilisé suivant l'une quelconque des revendications précédentes, dans lequel $R_1$ et $R_2$ sont choisis indépendamment entre l'hydrogène, un groupe méthyle et un radical bromo.

**7.** Composé destiné à être utilisé suivant l'une quelconque des revendications précédentes, dans lequel $R_3$ est l'hydrogène.

**8.** Composé destiné à être utilisé suivant la revendication 1, qui est
le [(1H-indole-3-yl)méthylène]carbazate de 1,1-dimétyléthyle (composé 4) ;
le [(1H-indole-3-yl)méthylène]carbazate d'éthyle (composé 12) ;
le [(1H-indole-3-yl)méthylène]hydrazide d'acide nicotinique (composé 13) ;
le [(5-bromo-1H-indole-3-yl)méthylène]carbazate d'éthyle (composé 52) ;
le [(1H-indole-3-yl)méthylène]hydrazide d'acide cyclobutanecarboxylique (composé 66) ;
le [(2-méthyl-1H-indole-3-yl)méthylène]carbazate d'éthyle (composé 67) ;
le [(2-méthyl-1H-indole-3-yl)méthyléne]hydrazide d'acide butanoïque (composé 69) ;
le [(2-méthyl-1H-indole-3-yl)méthylène]hydrazide d'acide propanoïque (composé 70) ;
le [(2-méthyl-1H-indole-3-yl)méthylène]hydrazide d'acide nicotinique (composé (96) ;
le [(2-méthyl-1H-indole-3-yl)méthylène]hydrazide de 1,1-diméthyléthyle (composé 97) ;
le [(1-acétylindole-3-yl)méthylène]hydrazide d'acide propanoïque (composé 145) ; ou
le [(2-méthyl-1H-indole-3-yl)méthylène]hydrazide d'acide phénylacétique (composé 167).

**9.** Composé destiné à être utilisé suivant la revendication 1, qui est le [(1-phénylméthylindole-3-yl)-propylidène]hydrazide d'acide acétique (composé 64).

10. Composé destiné à être utilisé suivant la revendication 1, qui est le [(1H-indole-3-yl)éthylidène]-carbazate d'éthyle (composé 79).

11. Composé destiné à être utilisé suivant la revendication 1, qui est le [1-(3-benzofurannyl)éthylidène]-hydrazide d'acide formique (composé 81).

12. Composé destiné à être utilisé suivant la revendication 1, qui est le [1-(3-benzothiényl)éthylidène]-hydrazide d'acide butanoïque (composé 118).

13. Composé destiné à être utilisé suivant la revendication 1, qui est le [1-(2-oxo-2H-1-benzopyranne-3-yl)-éthylidène]carbazate d'éthyle (composé 248) ; ou
le [1-(2-oxo-2H-1-benzopyranne-3-yl)éthylidène]hydrazide d'acide butanoïque (composé 250).

14. Acylhydrazone ou son hydrate ou son sel pharmaceutiquement acceptable, de formule définie suivant l'une quelconque des revendications précédentes, autre que
le [(1H-indole-3-yl)méthylène]hydrazide d'acide acétique (CA RN 1016-49-5) ;
le [(1H-indole-3-yl)méthylène]hydrazide d'acide 4-aminobenzoïque (CA RN 10245-42-8) ;
le [(1-méthylindole-2-yl)méthylène]hydrazide d'acide 4-méthoxybenzoique (CA RN 15565-86-3) ;
le (2-benzofurannylméthylène)hydrazide d'acide 4-morpholine-acétique (CA RN 72627-58-8) ;
le (1H-indole-3-ylméthylène)hydrazide d'acide (2-chlorophénoxy)acétique (CA RN 81111-25-3) ;
le (1H-indole-3-ylméthylène)hydrazide d'acide (4-chlorophénoxy)acétique (CA RN 81111-26-4) ;
le [(2-méthoxy-1H-indole-3-yl)méthylène]hydrazide d'acide (2-chlorophénoxy)acétique (CA RN 81122-74-9) ;
le [(2-méthoxy-1H-indole-3-yl)méthylène]hydrazide d'acide (4-chlorophénoxy)acétique (CA RN 81122-75-0) ;
le (1H-indole-3-ylméthylène)carbazate de méthyle (CA RN 88692-99-3) ;
le [1-(1H-indole-3-yl)propylidène]carbazate de phénylméthyle (CA RN 32947-16-3) ;
le [(2-méthyl-3-benzofurannyl)méthylène]hydrazide d'acide 1-pipéridine-acétique (CA RN 35806-44-1) ;
le (2-benzofurannylméthylène)hydrazide d'acide 1-pyrrolidine-acétique (CA RN 35806-70-3) ;
le (2-benzofurannylméthylène)hydrazide d'acide 1-pipéridine-acétique (CA RN 35806-85-0) ;
le [(2-méthyl-3-benzofurannyl)méthylène]hydrazide d'acide pyrrolidine-acétique (CA RN 35802-00-9) ;
le [(1H-indole-2-yl)méthylène]hydrazide d'acide formique (CA RN 50335-88-1) ;
le (1H-indole-2-ylméthylène)carbazate d'éthyle (CA RN 50335-89-2) ;
le [1-(2-benzofurannyl)éthylidène]carbazate d'éthyle (CA RN 56968-94-6) ;
le [1-(5-chloro-2-benzofurannyl)éthylidène]carbazate d'éthyle (CA RN 56968-95-7) ;
le (1H-indole-3-ylméthylène)carbazate de 1,1-diméthyléthyle (CA RN 57699-52-2) ;
le [(1H-indole-3-yl)méthylène]hydrazide d'acide 4-aminobenzoïque (CA RN 15641-21-1) ;
le [(1H-indole-3-yl)méthylène]hydrazide d'acide butyrique (CA RN 15641-24-4) ;
le [(1H-indole-3-yl)méthylène]hydrazide d'acide propionique (CA RN 15641-25-5) ;
le [(1H-indole-3-yl)méthylène]carbazate d'éthyle (CA RN 15641-27-7) ;
le [(1-méthylindole-2-yl)méthylène]hydrazide d'acide formique (CA RN 15641-28-8) ;
le [(1-méthylindole-2-yl)méthylène]hydrazide d'acide acétique (CA RN 15641-29-9) ;
le [(1-méthylindole-2-yl)méthylène]hydrazide d'acide propanoïque (CA RN 15641-30-2) ;
le [(1-méthylindole-2-yl)méthylène]hydrazide d'acide butanoïque (CA RN 15641-31-3) ;
le [(1-méthylindole-2-yl)méthylène]hydrazide d'acide 2-méthylpropanoïque (CA RN 15641-32-4) ;
le [(1-méthylindole-2-yl)méthylène]hydrazide d'acide phénylacétique (CA RN 15641-34-6) ;
le [(1-méthylindole-2-yl)méthylène]hydrazide d'acide 4-chlorobenzoïque (CA RN 15641-36-8) ;
le [(1-méthylindole-2-yl)méthylène]hydrazide d'acide 1-naphtalène-acétique (CA RN 15641-38-0) ;
le [(1-méthylindole-2-yl)méthylène]carbazate d'éthyle (CA RN 15565-88-5) ;
le [(1H-indole-3-yl)méthylène]hydrazide d'acide 4-chlorobenzoïque (CA RN 15641-01-7) ;
le [(1H-indole-3-yl)méthylène]hydrazide d'acide 2-chlorobenzoïque (CA RN 15641-03-9) ;
le [(1H-indole-3-yl)méthylène]hydrazide d'acide 3-chlorobenzoïque (CA RN 15641-04-0) ;
le [(1H-indole-3-yl)méthylène]hydrazide d'acide 2,4-dichlorobenzoïque (CA RN 15641-05-1) ;
le [(1H-indole-3-yl)méthylène]hydrazide d'acide 4-bromobenzoïque (CA RN 15641-06-2) ;
le [(1H-indole-3-yl)méthylène]hydrazide d'acide 2-méthylpropanoïque (CA RN 15641-12-0) ;
le [(1H-indole-3-yl)méthylène]hydrazide d'acide 4-méthoxybenzoïque (CA RN 15641-15-3) ;

le [(1H-indole-3-yl)méthylène]hydrazide d'acide phénylacétique (CA RN 15641-17-5) ;
le [(1H-indole-3-yl)méthylène]hydrazide d'acide 1-naphtalène-acétique (CA RN 15641-18-6) ;
le [(1H-indole-3-yl)méthylène]hydrazide d'acide 2-furoïque (CA RN 15641-19-7) ;
le [(1H-indole-3-yl)méthylène]hydrazide d'acide 2-naphtalène-acétique (CA RN 15718-86-2) ;
le [1-(1H-indole-3-yl)éthylidène]carbazate d'éthyle (CA RN 32927-03-0) ;
le 3-[(1H-indole-3-yl)méthylène]carbazate de phénylméthyle (CA RN 32927-05-2) ;
le [1-(1H-indole-3-yl)éthylidène]carbazate de phénylméthyle (CA RN 32927-06-3) ; ou
le [(1-méthylindole-3-yl)méthylène]carbazate d'éthyle (CA RN 32927-89-2).

**15.** Utilisation d'un composé suivant l'une quelconque des revendications précédentes pour la préparation d'un médicament destiné à détruire des vers parasites chez l'homme et les animaux à sang chaud domestiques de valeur.

**Revendications pour l'Etat contractant suivant : AT**

**1.** Procédé de préparation d'une acylhydrazone ou de son hydrate ou sel pharmaceutiquement acceptable, de formule

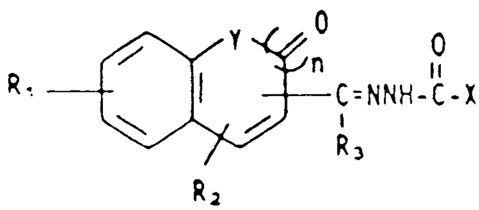

I

qui consiste (1) à faire réagir des composés correspondants de formules II et III

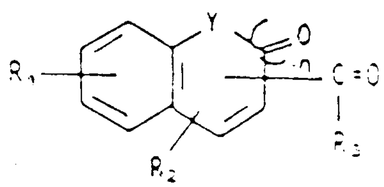

II

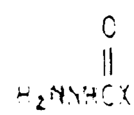

III

ou (2) à faire réagir le composé de formule II avec l'hydrazine, et l'hydrazone résultante avec un composé correspondant de formule WCOX ;

où W est un atome d'halogène, un groupe anhydride ou un autre groupe réactif ;

X représente (a) l'hydrogène ; (b) un groupe alkyle en $C_1$ à $C_{10}$ ; (c) un groupe alcényle en $C_2$ à $C_6$ ; (d) un groupe alcynyle en $C_2$ à $C_6$ ; (e) un groupe cycloalkyle en $C_3$ à $C_{10}$ facultativement substitué avec 1, 2 ou 3 radicaux alkyle en $C_1$ à $C_4$, alcényle en $C_2$ à $C_4$, alkoxy en $C_1$ à $C_4$, trifluorométhyle ou halogéno ; (f) un groupe pyrrolidinyle ; (g) un groupe pipéridinyle ; (h) un groupe 1-méthylpyrrolidinyle ; (i) un groupe 1-méthylpipéridinyle ; (j) un groupe alkoxyalkyle en $C_2$ à $C_6$ ; (k) un groupe (cycloalkyle en $C_3$ à $C_{10}$)-(alkyle en $C_1$ à $C_4$) ; (1) un groupe phényl-(alkyle en $C_1$ à $C_4$) facultativement substitué avec 1, 2 ou 3 radicaux halogéno ou alkoxy en $C_1$ à $C_4$, ou avec 1 ou 2 radicaux trifluorométhyle ; (m) un groupe phénoxy-(alkyle en $C_1$ à $C_4$) facultativement substitué avec 1, 2 ou 3 radicaux halogéno, ou avec 1 ou 2 radicaux trifluorométhyle ; (n) un groupe perhalogénalkyle en $C_1$ à $C_7$ ; (o) un groupe alkoxy en $C_1$ à $C_6$ ; (p) un groupe diphénylméthoxy ; (q) un groupe cycloalkyloxy en $C_3$ à $C_6$ facultativement substitué avec un ou deux radicaux alkyle en $C_1$ à $C_3$ ; (r) un groupe phénoxy facultativement substitué avec 1, 2 ou 3 radicaux halogéno ou avec 1 ou 2 radicaux trifluorométhyle ; (s) un groupe benzyloxy facultativement substitué avec 1, 2 ou 3 radicaux halogéno ou alkoxy en $C_1$ à $C_4$, ou avec 1 ou 2 radicaux trifluorométhyle ; (t) un groupe hétéro-aromatique facultativement substitué avec 1, 2 ou 3 radicaux halogéno, ou avec 1 ou 2 radicaux trifluorométhyle ;

(u) un groupe phényle substitué avec 1, 2 ou 3 radicaux halogéno, ou avec 1 ou 2 radicaux trifluorométhyle, amino, alkylamino en $C_1$ à $C_3$, di(alkyle en $C_1$ à $C_3$)amino, alkyle en $C_1$ à $C_5$ ou alkoxy en $C_1$ à $C_3$ ; (v) un groupe N-morpholinyl-(alkyle en $C_1$ à $C_4$) ; (w) un groupe N-pipéridinyl-(alkyle en $C_1$ à $C_4$) ; (x) un groupe N-pyrrolidinyl-(alkyle en $C_1$ à $C_4$) ; (y) des substituants hydrocarbonés polycycliques pontés de 6 à 10 atomes cycliques, facultativement substitués avec 1, 2 ou 3 radicaux alkyle en $C_1$ à $C_3$ : ou bien (z) un groupe naphtyl(alkyle en $C_1$ à $C_3$) facultativement substitué avec 1, 2 ou 3 radicaux alkyle en $C_1$ à $C_4$, alkoxy en $C_1$ à $C_4$, halogéno ou trifluorométhyle ;

Y est un atome d'oxygène, un atome de soufre ou un groupe N-Z ;

Z est un atome d'hydrogène ; un groupe alkyle en $C_1$ à $C_4$ ; acétyle ; benzoyle ; phényle facultativement substitué avec 1, 2 ou 3 radicaux alkyle en $C_1$ à $C_4$, alkoxy en $C_1$ à $C_3$, halogéno, trifluorométhyle, dialkylamino en $C_2$ à $C_6$, alkylthio en $C_1$ à $C_3$, nitro ou phénoxy facultativement substitué avec 1, 2 ou 3 radicaux alkyle en $C_1$ à $C_4$, alkoxy en $C_1$ à $C_3$, halogéno ou trifluorométhyle ; ou un groupe phényl-(alkyle en $C_1$ à $C_4$) facultativement substitué avec un, 2 ou 3 radicaux alkyle en $C_1$ à $C_4$, alkoxy en $C_1$ à $C_4$, halogéno ou trifluorométhyle ;

$R_1$ est l'hydrogène ; un groupe hydroxy ; un groupe alkyle en $C_1$ à $C_4$ ; un groupe alkoxy en $C_1$ à $C_3$ ; un groupe alkylthio en $C_1$ à $C_3$ ; un radical halogéno ; un groupe nitro ; benzyloxy ; ou trifluorométhyle ;

$R_2$ est l'hydrogène ; un groupe hydroxy ; alkyle en $C_1$ à $C_4$ ; alkoxy en $C_1$ à $C_3$ ; alkylthio en $C_1$ à $C_3$ ; un radical halogéno ou un groupe trifluorométhyle ;

$R_3$ est l'hydrogène ; un groupe alkyle en $C_1$ à $C_4$ ; un groupe cycloalkyle en $C_3$ à $C_6$ facultativement substitué avec 1, 2 ou 3 radicaux alkyle en $C_1$ à $C_3$ ; un groupe phényle facultativement substitué avec 1, 2 ou 3 radicaux alkyle en $C_1$ à $C_4$, halogéno, trifluorométhyle ou alkoxy en $C_1$ à $C_3$ ; un groupe phényl-(alkyle en $C_1$ à $C_3$) facultativement substitué avec 1, 2 ou 3 radicaux alkyle en $C_1$ à $C_4$, halogéno, trifluorométhyle ou alkoxy en $C_1$ à $C_3$ ; ou un groupe 1,3-dioxacyclohexane-5-yle ; et

$\underline{n}$ a la valeur 0 ou 1 ;

sous réserve que

(i) $\underline{n}$ soit égal à 0 lorsque Y est un atome de soufre ou un groupe N-Z ;

(ii) lorsque X est un groupe n-propyle, $R_3$ est un groupe méthyle, $R_1$ et $R_2$ sont de l'hydrogène, le composé soit autre chose qu'une indole-3-ylacylhydrazone ;

(iii) lorsque X est un groupe cyclopropyle, $R_2$ est un groupe 2-méthyle, $R_1$ et $R_3$ sont de l'hydrogène, le composé soit autre chose qu'une indole-3-ylacylhydrazone ;

(iv) lorsque X est un groupe cyclohexylméthyle, $R_1$ et $R_2$ sont de l'hydrogène, $R_3$ est un groupe éthyle, le composé soit autre chose que la 1-benzylindole-3-yl-acylhydrazone ;

(v) lorsque X est un groupe tertio-butoxy, le composé soit une 2-benzofurannylacylhydrazone et que lorsque $R_1$ et $R_2$ sont de l'hydrogène, $R_3$ soit autre chose qu'un groupe méthyle ;

(vi) lorsque X est un groupe éthoxy, $R_1$, $R_2$ et $R_3$ sont de l'hydrogène, le composé soit autre chose qu'une 1-acétylindole-3-ylacylhydrazone ;

(vii) lorsque X est un groupe éthoxy, $R_1$ et $R_2$ sont de l'hydrogène, $R_3$ est un groupe éthyle, le composé soit autre chose qu'une 1-benzylindole-3-ylacylhydrazone ;

(viii) lorsque X est un groupe éthoxy, $R_1$ et $R_2$ sont de l'hydrogène, $R_3$ est un groupe méthyle, le composé soit autre chose qu'une 3-benzothiofuranneacylhydrazone ;

(ix) lorsque X est un groupe phénoxy, $R_1$ et $R_2$ sont de l'hydrogène et $R_3$ est un groupe méthyle, le composé soit autre chose qu'une 2-benzofurannylacylhydrazone ;

(x) lorsque X est un groupe benzyloxy et $R_1$, $R_2$ et $R_3$ sont de l'hydrogène, le composé soit autre chose qu'une indole-3-ylacylhydrazone ;

(xi) lorsque X est un groupe 4-éthyloxyphényle ou 4-chlorophényle, $R_1$ et $R_2$ sont de l'hydrogène, $R_3$ est un groupe méthyle, le composé soit autre chose qu'une 3-benzothiénylacylhydrazone ;

(xii) lorsque X est un groupe 4-chlorophényle, $R_1$ et $R_2$ sont de l'hydrogène, $R_3$ est un groupe éthyle, le composé soit autre chose qu'une 1-benzylindole-3-ylacylhydrazone ;

(xiii) lorsque X est un groupe 4-méthylphényle, 3,4-diméthoxyphényle ou 3-méthoxyphényle et $R_1$, $R_2$ et $R_3$ sont de l'hydrogène, le composé soit autre chose qu'une indole-3-ylacylhydrazone ;

(xiv) lorsque X est un groupe 4-éthoxyphényle, $R_1$, $R_2$ et $R_3$ sont de l'hydrogène, le composé soit autre chose qu'une 1-acétylindole-3-ylacylhydrazone ;

(xv) lorsque $R_1$ est un groupe benzyloxy, X soit autre chose qu'un groupe alkyle ; et

(xvi) l'acylhydrazone ou son hydrate ou sel pharmaceutiquement acceptable est autre chose que

le [(1H-indole-3-yl)méthylène]hydrazide d'acide acétique (CA RN 1016-49-5) ;

le [(1H-indole-3-yl)méthylène]hydrazide d'acide 4-aminobenzoïque (CA RN 10245-42-8) ;

le [(1-méthylindole-2-yl)méthylène]hydrazide d'acide 4-méthoxybenzoïque (CA RN 15565-86-3) ;

le (2-benzofurannylméthylène)hydrazide d'acide 4-morpholine-acétique (CA RN 72627-58-8) ;
le (1H-indole-3-ylméthylène)hydrazide d'acide (2-chlorophénoxy)acétique (CA RN 81111-25-3) ;
le (1H-indole-3-ylméthylène)hydrazide d'acide (4-chlorophénoxy)acétique (CA RN 81111-26-4) ;
le [(2-méthoxy-1H-indole-3-yl)méthylène]hydrazide d'acide (2-chlorophénoxy)acétique (CA RN 81122-74-9) ;
le [(2-méthoxy-1H-indole-3-yl)méthylène]hydrazide d'acide (4-chlorophénoxy)acétique (CA RN 81122-75-0) ;
le (1H-indole-3-ylméthylène)carbazate de méthyle (CA RN 88692-99-3) ;
le [1-(1H-indole-3-yl)propylidène]carbazate de phénylméthyle (CA RN 32947-16-3) ;
le [(2-méthyl-3-benzofurannyl)méthylène]hydrazide d'acide 1-pipéridine-acétique (CA RN 35806-44-1) ;
le (2-benzofurannylméthylène)hydrazide d'acide 1-pyrrolidine-acétique (CA RN 35806-70-3) ;
le (2-benzofurannylméthylène)hydrazide d'acide 1-pipéridine-acétique (CA RN 35806-85-0) ;
le [(2-méthyl-3-benzofurannyl)méthylène]hydrazide d'acide pyrrolidine-acétique (CA RN 35802-00-9) ;
le [(1H-indole-2-yl)méthylène]hydrazide d'acide formique (CA RN 50335-88-1) ;
le (1H-indole-2-ylméthylène)carbazate d'éthyle (CA RN 50335-89-2) ;
le [1-(2-benzofurannyl)éthylidène]carbazate d'éthyle (CA RN 56968-94-6) ;
le [1-(5-chloro-2-benzofurannyl)éthylidène]carbazate d'éthyle (CA RN 56968-95-7) ;
le (1H-indole-3-ylméthylène)carbazate de 1,1-diméthyléthyle (CA RN 57699-52-2) ;
le [(1H-indole-3-yl)méthylène]hydrazide d'acide 4-aminobenzoïque (CA RN 15641-21-1) ;
le [(1H-indole-3-yl)méthylène]hydrazide d'acide butyrique (CA RN 15641-24-4) ;
le [(1H-indole-3-yl)méthylène]hydrazide d'acide propionique (CA RN 15641-25-5) ;
le [(1H-indole-3-yl)méthylène] carbazate d'éthyle (CA RN 15641-27-7) ;
le [(1-méthylindole-2-yl)méthylène]hydrazide d'acide formique (CA RN 15641-28-8) ;
le [(1-méthylindole-2-yl)méthylène]hydrazide d'acide acétique (CA RN 15641-29-9) ;
le [(1-méthylindole-2-yl)méthylène]hydrazide d'acide propanoïque (CA RN 15641-30-2) ;
le [(1-méthylindole-2-yl)méthylène]hydrazide d'acide butanoïque (CA RN 15641-31-3) ;
le [(1-méthylindole-2-yl)méthylène]hydrazide d'acide 2-méthylpropanoïque (CA RN 15641-32-4) ;
le [(1-méthylindole-2-yl)méthylène]hydrazide d'acide phénylacétique (CA RN 15641-34-6) ;
le [(1-méthylindole-2-yl)méthylène]hydrazide d'acide 4-chlorobenzoïque (CA RN 15641-36-8) ;
le [(1-méthylindole-2-yl)méthylène]hydrazide d'acide 1-naphtalène-acétique (CA RN 15641-38-0) ;
le [(1-méthylindole-2-yl)méthylène]carbazate d'éthyle (CA RN 15565-88-5) ;
le [(1H-indole-3-yl)méthylène]hydrazide d'acide 4-chlorobenzoïque (CA RN 15641-01-7) ;
le [(1H-indole-3-yl)méthylène]hydrazide d'acide 2-chlorobenzoïque (CA RN 15641-03-9) ;
le [(1H-indole-3-yl)méthylène]hydrazide d'acide 3-chlorobenzoïque (CA RN 15641-04-0) ;
le [(1H-indole-3-yl)méthylène]hydrazide d'acide 2,4-dichlorobenzoïque (CA RN 15641-05-1) ;
le [(1H-indole-3-yl)méthylène]hydrazide d'acide 4-bromobenzoïque (CA RN 15641-06-2) ;
le [(1H-indole-3-yl)méthylène]hydrazide d'acide 2-méthylpropanoïque (CA RN 15641-12-0) ;
le [(1H-indole-3-yl)méthylène]hydrazide d'acide 4-méthoxybenzoïque (CA RN 15641-15-3) ;
le [(1H-indole-3-yl)méthylène]hydrazide d'acide phénylacétique (CA RN 15641-17-5) ;
le [(1H-indole-3-yl)méthylène]hydrazide d'acide 1-naphtalène-acétique (CA RN 15641-18-6) ;
le [(1H-indole-3-yl)méthylène]hydrazide d'acide 2-furoïque (CA RN 15641-19-7) ;
le [(1H-indole-3-yl)méthylène]hydrazide d'acide 2-naphtalène-acétique (CA RN 15718-86-2) ;
le [1-(1H-indole-3-yl)éthylidène]carbazate d'éthyle (CA RN 32927-03-0) ;
le 3-[(1H-indole-3-yl)méthylène]carbazate de phénylméthyle (CA RN 32927-05-2) ;
le [1-(1H-indole-3-yl)éthylidène]carbazate de phénylméthyle (CA RN 32927-06-3) ; ou
le [(1-méthylindole-3-yl)méthylène]carbazate d'éthyle (CA RN 32927-89-2).

**2.** Procédé suivant la revendication 1, dans lequel n est égal à 0.

**3.** Procédé suivant la revendication 2, dans lequel Y est le groupe NH.

**4.** Procédé suivant la revendication 3, dans lequel l'acylhydrazone est une 1H-indole-3-ylacylhydrazone.

**5.** Procédé suivant l'une quelconque des revendications précédentes, dans lequel X est un groupe

cyclobutyle, tertio-butyoxy, éthoxy, éthyle ou 3-pyridinyle.

6. Procédé suivant l'une quelconque des revendications précédentes, dans lequel $R_1$ et $R_2$ sont choisis indépendamment entre l'hydrogène, le groupe méthyle et le radical bromo.

7. Procédé suivant l'une quelconque des revendications précédentes, dans lequel $R_3$ est l'hydrogène.

8. Procédé suivant la revendication 1, dans lequel l'acylhydrazone est
le [(1H-indole-3-yl)méthylène]carbazate de 1,1-diméthyléthyle (composé 4) ;
le [(1H-indole-3-yl)méthylène]hydrazide d'acide nicotinique (composé 13) ;
le [(5-bromo-1H-indole-3-yl)méthylène]carbazate d'éthyle (composé 52) ;
le [(1H-indole-3-yl)méthylène]hydrazide d'acide cyclobutanecarboxylique (composé 66) ;
le [(2-méthyl-1H-indole-3-yl)méthylène]carbazate d'éthyle (composé 67) ;
le [(2-méthyl-1H-indole-3-yl)méthylène]hydrazide d'acide butanoïque (composé 69) ;
le [(2-méthyl-1H-indole-3-yl)méthylène]hydrazide d'acide propanoïque (composé 70) ;
le [(2-méthyl-1H-indole-3-yl)méthylène]hydrazide d'acide nicotinique (composé (96) ;
le [(2-méthyl-1H-indole-3-yl)méthylène]hydrazide de 1,1-diméthyléthyle (composé 97) ;
le [(1-acétylindole-3-yl)méthylène]hydrazide d'acide propanoïque (composé 145) ; ou
le [(2-méthyl-1H-indole-3-yl)méthylène]hydrazide d'acide phénylacétique (composé 167).

9. Procédé suivant la revendication 1, dans lequel l'acylhydrazone est le [(1-phénylméthylindole-3-yl)-propylidène]hydrazide d'acide acétique (composé 64).

10. Procédé suivant la revendication 1, dans lequel l'acylhydrazone est le [1-(3-benzofurannyl)éthylidène]-hydrazide d'acide formique (composé 81).

11. Procédé suivant la revendication 1, dans lequel l'acylhydrazone est le [1-(3-benzothiényl)éthylidène]-hydrazide d'acide butanoïque (composé 118).

12. Procédé suivant la revendication 1, dans lequel l'acylhydrazone est le [1-(2-oxo-2H-1-benzopyranne-3-yl)éthylidène]carbazate d'éthyle (composé 248) ; ou
le [1-(2-oxo-2H-1-benzopyranne-3-yl)éthylidène]hydrazide d'acide butanoïque (composé 250).

**Patentansprüche**
**Patentansprüche für folgende Vertragsstaaten : BE, CH, DE, FR, GB, IT, LU, NL, SE**

1. Verbindung zur Verwendung beim Abtöten von parasitischen Würmern bei Menschen und wertvollen, warmblütigen Haustieren, welche ein Acylhydrazon oder Hydrat oder ein pharmazeutisch verträgliches Salz davon der Formel

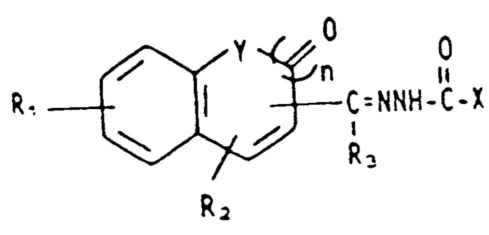

I

darstellt, worin X (a) Wasserstoff ist; (b) $C_1$-$C_{10}$Alkyl bedeutet; (c) $C_2$-$C_6$Alkenyl bedeutet; (d) $C_2$-$C_6$Alkinyl bedeutet; (e) Cyclo($C_3$-$C_{10}$)alkyl bedeutet, welches gegebenenfalls mit einem, zwei oder drei $C_1$-$C_4$Alkyl, $C_2$-$C_4$Alkenyl, $C_1$-$C_4$Alkoxy, Trifluormethyl oder Halogenen substituiert ist, (f) Pyrrolidinyl bedeutet; (g) Piperidinyl bedeutet; (h) 1-Methylpyrrolidinyl bedeutet; (i) 1-Methylpiperidinyl bedeutet; (j) $C_2$-$C_6$Alkoxyalkyl bedeutet; (k) Cyclo($C_3$-$C_{10}$)alkyl($C_1$-$C_4$)alkyl bedeutet; (1) Phenyl($C_1$-$C_4$)-alkyl bedeutet, welches gegebenenfalls mit einem, zwei oder drei Halogenen oder $C_1$-$C_4$Alkoxy oder einer oder zwei Trifluormethylgruppen substituiert ist; (m) Phenoxy($C_1$-$C_4$)alkyl bedeutet, gegebenenfalls mit einem, zwei oder drei Halogenen oder einer oder zwei Trifluormethylgruppen substituiert ist; (n) Perhalo($C_1$-$C_7$)alkyl bedeutet; (o) $C_1$-$C_6$Alkoxy bedeutet; (p) Diphenylmethoxy bedeutet; (q) Cyclo($C_3$-

$C_6$)alkoxy bedeutet, welches gegebenenfalls mit einem oder zwei Alkylen substituiert ist; (r) Phenoxy bedeutet, gegebenenfalls substituiert mit einem, zwei oder drei Halogenen oder einer oder zwei Trifluormethylgruppen; (s) Benzyloxy bedeutet, welches gegebenenfalls mit einem, zwei oder drei Halogenen oder $C_1$-$C_4$Alkoxygruppen oder einer oder zwei Trifluormethylgruppen substituiert ist; (t) heteroaromatische Gruppen, welche gegebenenfalls mit einem, zwei oder drei Halo-genen oder einer oder zwei Trifluormethylgruppen substituiert ist; (u) Phenyl bedeutet, gegebenenfalls mit einem, zwei oder drei Halogenen oder einer oder zwei Trifluormethylgruppen, Amino, Alkyl($C_1$-$C_3$)amino, Dialkyl($C_1$-$C_3$)amino, $C_1$-$C_5$Alkyl oder $C_1$-$C_3$-Alkoxy substituiert ist; (v) N-Morpholinyl($C_1$-$C_4$)alkyl bedeutet; (w) N-Piperidinyl($C_1$-$C_4$)alkyl bedeutet; (x) N-Pyrro-lidinyl($C_1$-$C_4$)alkyl bedeutet; (y) überbrückte polycyclische Kohlenwasserstoff-Substituenten von sechs bis zehn Kohlenstoffen bedeutet, welche gegebenenfalls mit einer, zwei oder drei ($C_1$-$C_3$)Alkylgruppen substituiert sind; oder (z) Naphthyl($C_1$-$C_3$)alkyl bedeutet, welches gegebenenfalls mit einem, zwei oder drei $C_1$-$C_4$Alkyl, $C_1$-$C_4$Alkoxy, Halogenen oder Trifluormethyl substituiert ist;

worin Y ein Sauerstoffatom, ein Schwefelatom oder eine N-Z-Gruppe bedeutet;

worin Z Wasserstoff, $C_1$-$C_4$Alkyl; Acetyl, Benzoyl, Phenyl, gegebenenfalls substituiert mit einem, zwei oder drei $C_1$-$C_4$Alkyl, $C_1$-$C_3$Alkoxy, Halogen, Trifluormethyl, $C_2$-$C_6$Di-alkylamino, $C_1$-$C_3$Alkylthio, Nitro oder Phenoxy, welches gegebenenfalls mit einem, zwei oder drei $C_1$-$C_4$Alkyl, $C_1$-$C_3$Alkoxy, Halogen oder Trifluormethyl substituiert ist; oder Phenyl($C_1$-$C_4$)alkyl, gegebenenfalls substituiert mit einem, zwei oder drei $C_1$-$C_4$Alkyl, $C_1$-$C_4$Alkoxy, Halogen oder Trifluormethyl bedeutet;

worin $R_1$ Wasserstoff, Hydroxy, $C_1$-$C_4$Alkyl, $C_1$-$C_3$Alkoxy, $C_1$-$C_3$Alkylthio, Halogen, Nitro, Benzyloxy oder Trifluormethyl bedeutet;

worin $R_2$ Wasserstoff, Hydroxy, $C_1$-$C_4$Alkyl, $C_1$-$C_3$Alkoxy, $C_1$-$C_3$Alkylthio, Halogen oder Trifluormethyl bedeutet, worin $R_3$ Wasserstoff, $C_1$-$C_4$Alkyl, Cyclo($C_3$-$C_6$)alkyl, gegebenenfalls substituiert mit einem, zwei oder drei $C_1$-$C_3$Alkyl, Phenyl, gegebenenfalls substituiert mit einem, zwei oder drei $C_1$-$C_4$Alkyl, Halogen, Trifluormethyl oder $C_1$-$C_3$Alkoxy, Phenyl($C_1$-$C_3$)alkyl, gegebenenfalls substituiert mit einem, zwei oder drei $C_1$-$C_4$Alkyl, Halogen, Trifluormethyl oder $C_1$-$C_3$Alkoxy oder 1,3-Dioxacyclohexan-5-yl bedeutet, und

n 0 oder 1 bedeutet,

mit den Provisos, daß

(i) n ist 0, wenn Y ein Schwefelatom oder eine N-Z-Gruppe bedeutet;

(ii) wenn X N-Propyl bedeutet, $R_3$ Methyl, $R_1$ und $R_2$ Wasserstoff bedeuten, die Verbindung etwas Anderes als ein Indol-3-yl-acylhydrazon ist;

(iii) wenn X Cyclopropyl, $R_2$ 2-Methyl, $R_1$ und $R_3$ Wasserstoff bedeuten, die Verbindung etwas Anderes als ein Indol-3-yl-acylhydrazon bedeutet;

(iv) wenn X Cyclohexylmethyl, $R_1$ und $R_2$ Wasserstoff, $R_3$ Ethyl bedeuten, bedeutet die Verbindung etwas Anderes als 1-Benzylindol-3-yl-acylhydrazon;

(v) wenn X t-Butoxy bedeutet, ist die Verbindung ein 2-Benzofuranylacylhydrazon, und $R_1$ und $R_2$ sind Wasserstoff und $R_3$ etwas Anderes als Methyl;

(vi) wenn X Ethoxy bedeutet, $R_1$, $R_2$ und $R_3$ Wasserstoff sind, ist die Verbindung etwas Anderes als 1-Acetylindol-3-yl-acylhydrazon;

(vii) wenn X Ethoxy ist, $R_1$ und $R_2$ Wasserstoff sind, $R_3$ Ethyl ist, ist die Verbindung etwas Anderes als 1-Benzylindol-3-yl-acylhydrazon;

(viii) wenn X Ethoxy ist, $R_1$ und $R_2$ Wasserstoff sind, $R_3$ Methyl ist, ist die Verbindung etwas Anderes als ein 3-Benzothiofuran-acylhydrazon;

(ix) wenn X Phenoxy ist, $R_1$ und $R_2$ Wasserstoff sind und $R_3$ Methyl ist, ist die Verbindung etwas Anderes als ein 2-Benzofuranyl-acylhydrazon;

(x) wenn X Benzyloxy ist und $R_1$, $R_2$ und $R_3$ Wasserstoff sind, ist die Verbindung etwas Anderes als ein Indol-3-yl-acylhydrazon;

(xi) wenn X 4-Ethoxyphenyl oder 4-Chlorphenyl ist, $R_1$ und $R_2$ Wasserstoff sind und $R_3$ Methyl ist, ist die Verbindung etwas Anderes als ein 3-Benzothienyl-acylhydrazon;

(xii) wenn X 4-Chlorphenyl ist, $R_1$ und $R_2$ Wasserstoff sind, $R_3$ Ethyl ist, ist die Verbindung etwas Anderes als 1-Benzylindol-3-yl-acylhydrazon;

(xiii) wenn X 4-Methylphenyl, 3,4-Dimethoxyphenyl oder 3-Methoxyphenyl ist und $R_1$, $R_2$ und $R_3$ Wasserstoff sind, ist die Verbindung etwas Anderes als ein Indol-3-yl-acylhydrazon;

(xiv) wenn X 4-Ethoxyphenyl ist, $R_1$, $R_2$ und $R_3$ Wasserstoff sind, ist die Verbindung etwas Anderes als 1-Acetylindol-3-yl-acylhydrazon; und (xv) wenn $R_1$ Benzyloxy ist, ist X etwas Anderes als Alkyl.

**2.** Verbindung zur Verwendung nach Anspruch 1, worin n 0 ist.

3. Verbindung zur Verwendung nach Anspruch 2, worin Y NH bedeutet.

4. Verbindung zur Verwendung nach Anspruch 3, welche ein 1H-Indol-3-yl-acylhydrazon ist.

5. Verbindung zur Verwendung nach einem der vorhergehenden Ansprüche, worin X Cyclobutyl, t-Butoxy, Ethoxy, Ethyl oder 3-Pyridinyl bedeutet.

6. Verbindung zur Verwendung nach einem der vorhergehenden Ansprüche, worin $R_1$ und $R_2$ unabhängig voneinander aus Wasserstoff, Methyl und Brom gewählt werden.

7. Verbindung zur Verwendung nach einem der vorhergehenden Ansprüche, worin $R_3$ Wasserstoff ist.

8. Verbindung zur Verwendung nach Anspruch 1, welche 1,1-Dimethylethyl-[(1H-indol-3-yl)methylen]-carbazat (Verb.4); Ethyl-[(1H-indol-3-yl)methylen]carbazat (Verb.12); Nicotinsäure-[(1H-indol-3-yl)-methylen]hydrazid (Verb.13); Ethyl-[(5-brom-1H-indol-3-yl)methylen]carbazat (Verb.52);
Cyclobutancarbonsäure-[(1H-indol-3-yl)methylen]hydrazid (Verb.66);
Ethyl-[(2-methyl-1H-indol-3-yl)methylen]carbazat (Verb.67); Butansäure[(2-methyl-1H-indol-3-yl)-methylen]hydrazid (Verb.69);
Propansäure-[(2-methyl-1H-indol-3-yl)methylen]hydrazid (Verb.70);
Nicotinsäure-[(2-methyl-1H-indol-3-yl)methylen]hydrazid (Verb.96);
1,1-Dimethylethyl-[(2-methyl-1H-indol-3-yl)methylen]hydrazid (Verb.97);
Propansäure-[(1-acetylindol-3-yl)methylen]hydrazid (Verb.145); oder
Phenylessigsäure-[(2-methyl-1H-indol-3-yl)methylen]hydrazid (Verb.167),
ist.

9. Verbindung zur Verwendung nach Anspruch 1, welche Essigsäure-[(1-phenylmethylindol-3-yl)-propyliden]hydrazid (Verb.64) ist.

10. Verbindung zur Verwendung nach Anspruch 1, welche Ethyl-[(1H-indol-3-yl)ethyliden]carbazat (Verb.79) ist.

11. Verbindung zur Verwendung nach Anspruch 1, welche Ameisensäure-[1-(3-benzofuranyl)ethyliden]-hydrazid (Verb.81) ist.

12. Verbindung zur Verwendung nach Anspruch 1, welche Buttersäure-[1-(3-benzothienyl)ethyliden]-hydrazid (Verb.118) ist.

13. Verbindung zur Verwendung nach Anspruch 1, welche Ethyl[1-(2-oxo-2H-1-benzopyran-3-yl)ethyliden]-carbazat (Verb.248); oder
Buttersäure-[1-(2-oxo-2H-1-benzopyran-3-yl)ethyliden]hydrazid (Verb.250) ist.

14. Ein Acylhydrazon oder Hydrat oder pharmazeutisch verträgliches Salz davon der in einem der vorhergehenden Ansprüche definierten Formel, welches nicht
Essigsäure-[(1H-indol-3-yl)methylen]hydrazid, (CA-RN-1016-49-5);
4-Aminobenzoesäure-[(1H-indol-3-yl)methylen]hydrazid, (CA-RN 10245-42-8);
4-Methoxybenzoesäure-[(1-methylindol-2-yl)methylen]hydrazid, (CA RN 15565-86-3);
4-Morpholinessigsäure-(2-benzofuranylmethylen)hydrazid, (CA-RN 72627-58-8);
(2-Chlorphenoxy)essigsäure (1H-indol-3-yl-methylen)hydrazid, (CA-RN 81111-25-3);
(4-Chlorphenoxy)essigsäure-(1H-indol-3-yl-methylen)hydrazid, (CA-RN 81111-26-4);
(2-Chlorphenoxy)essigsäure-[(2-methoxy-1H-indol-3-yl)methylen]hydrazid, (CA-RN 81122-74-9);
(4-Chlorphenoxy)essigsäure-[(2-methoxy-1H-indol-3-yl)methylen]hydrazid (CA-RN 8122-75-0);
Methyl-(1H-indol-3-yl-methylen)carbazat (CA-RN 88692-99-3);
Phenylmethyl-[1-(1H-indol-3-yl)propyliden]carbazat (CA-RN 32947-16-3);
1-Piperidinessigsäure-[(2-methyl-3-benzofuranyl)methylen]hydrazid (CA RN 35806-44-1);
1-Pyrrolidinessigsäure-(2-benzofuranylmethylen)hydrazid (CA RN 35806-70-3);
1-Piperidinessigsäure-(2-benzofuranylmethylen)hydrazid (CA RN 35806-85-0);
1-Pyrrolidinessigsäure-[(2-methyl-3-benzofuranyl)methylen]hydrazid (CA RN 35802-00-9);
Ameisensäure-[(1H-indol-2-yl)-methylen]hydrazid (CA RN 50335-88-1);

Ethyl-(1H-indol-2-yl-methylen)carbazat (CA RN 50335-89-2); Ethyl-[1-(2-benzofuranyl)ethyliden]carbazat (CA RN 56968-94-6);
Ethyl-[1-(5-chlor-2-benzofuranyl)ethyliden]carbazat (CA RN 56968-95-7);
1,1-Dimethylethyl-(1H-indol-3-yl-methylen)carbazat (CA RN 57699-52-2);
4-Aminobenzoesäure-[(1H-indol-3-yl)methylen]hydrazid (CA RN 15641-21-1);
Buttersäure-[(1H-indol-3-yl)methylen]hydrazid (CA RN 15641-24-4);
Propionsäure-[(1H-indol-3-yl)methylen]hydrazid (CA RN 15641-25-5);
Ethyl-[(1H-indol-3-yl)methylen]carbazat (CA RN 15641-27-7);
Ameisensäure-[(1-methylindol-2-yl)methylen]hydrazid (CA RN 15641-28-8);
Essigsäure-[(1-methylindol-2-yl)methylen]hydrazid (CA RN 15641-29-9);
Propansäure-[(1-methylindol-2-yl)methylen]hydrazid (CA RN 15641-30-2);
Butansäure-[(1-methylindol-2-yl)methylen]hydrazid (CA RN 15641-31-3);
2-Methylpropansäure-[(1-methylindol-2-yl)methylen]hydrazid (CA RN 15641-32-4);
Phenylessigsäure-[(1-methylindol-2-yl)methylen]hydrazid (CA RN 15641-34-6);
4-Chlorbenzoesäure-[(1-methylindol-2-yl)methylen]hydrazid (CA RN 15641-36-8);
1-Naphthalinessigsäure-[(1-methylindol-2-yl)methylen]hydrazid (CA RN 15641-38-0);
Ethyl-[(1-methylindol-2-yl)methylen]carbazat (CA RN 15565-88-5);
4-Chlorbenzoesäure-[(1H-indol-3-yl)methylen]hydrazid (CA RN 15641-01-7);
2-Chlorbenzoesäure-[5(1H-indol-3-yl)methylen]hydrazid (CA RN 15641-03-9);
3-Chlorbenzoesäure-[(1H-indol-3-yl)methylen]hydrazid (CA RN 15641-04-0);
2,4-Dichlorbenzoesäure-[(1H-indol-3-yl)methylen]hydrazid (CA RN 15641-05-1);
4-Brombenzoesäure-[(1H-indol-3-yl)methylen]hydrazid (CA RN 15641-06-2);
2-Methylpropansäure-[(1H-indol-3-yl)methylen]hydrazid (CA RN 15641-12-0);
4-Methoxybenzoesäure-[(1H-indol-3-yl)methylen]hydrazid (CA RN 15641-15-3);
Phenylessigsäure-[(1H-indol-3-yl)methylen]hydrazid (CA RN 15641-17-5);
1-Naphthalinessigsäure-[(1H-indol-yl)methylen]hydrazid (CA RN 15641-18-6);
2-Furonsäure-[(1H-indol-3-yl)methylen]hydazid (CA RN 15641-19-7);
2-Naphthalinessigsäure-[(1H-indol-3-yl)methylen]hydrazid (CA RN 15718-86-2);
Ethyl-[1-(1H-indol-3-yl)ethyliden]carbazat (CA RN 32927-03-0); Phenylmethyl-3-[(1H-indol-3-yl)-methylen]carbazat (CA RN 32927-05-2);
Phenylmethyl-[1-(1H-indol-3-yl)ethyliden]carbazat (CA RN 32927-06-3); oder
Ethyl-[(1-methylindol-3-yl)methylen]carbazat (CA RN 32927-89-2),
ist.

**15.** Verwendung einer Verbindung nach einem der vorhergehenden Ansprüche zur Herstellung eines Medikaments zur Verwendung bei der Abtötung von parasitischen Würmern bei Menschen und wertvollen, warmblütigen Haustieren.

**Patentansprüche für folgende Vertragsstaat : AT**

**1.** Verfahren zur Herstellung eines Acylhydrazons oder Hydrates oder pharmazeutisch verträglichen Salzes davon der Formel

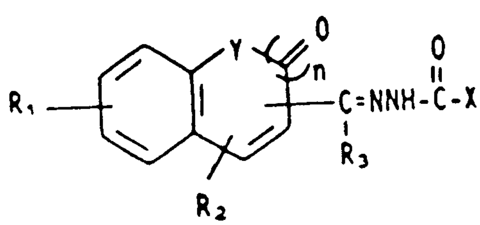

I

welches entweder (1) die Umsetzung der entsprechenden Verbindungen der Formeln II und III

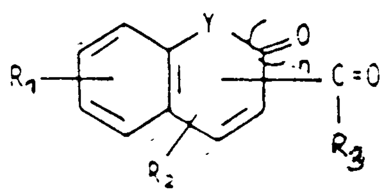

II

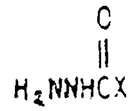

III

oder (2) Umsetzen der Verbindung der Formel II mit Hydrazin und des resultierenden Hydrazons mit einer entsprechenden Verbindung der Formel WCOX umfaßt; worin W ein Halogenatom, ein Anhydrid oder eine andere reaktive Gruppe darstellt; worin X (a) Wasserstoff ist; (b) $C_1$-$C_{10}$Alkyl bedeutet; (c) $C_2$-$C_6$Alkenyl bedeutet; (d) $C_2$-$C_6$Alkinyl bedeutet; (e) Cyclo($C_3$-$C_{10}$)alkyl bedeutet, welches gegebenenfalls mit einem, zwei oder drei $C_1$-$C_4$Alkyl, $C_2$-$C_4$Alkenyl, $C_1$-$C_4$Alkoxy, Trifluormethyl oder Halogenen substituiert ist, (f) Pyrrolidinyl bedeutet; (g) Piperidinyl bedeutet; (h) 1-Methylpyrrolidinyl bedeutet; (i) 1-Methylpiperidinyl bedeutet; (j) $C_2$-$C_6$Alkoxyalkyl bedeutet; (k) Cyclo($C_3$-$C_{10}$)alkyl($C_1$-$C_4$)alkyl bedeutet; (1) Phenyl($C_1$-$C_4$)-alkyl bedeutet, welches gegebenenfalls mit einem, zwei oder drei Halogenen oder $C_1$-$C_4$Alkoxy oder einer oder zwei Trifluormethylgruppen substituiert ist; (m) Phenoxy($C_1$-$C_4$)alkyl bedeutet, gegebenenfalls mit einem, zwei oder drei Halogenen oder einer oder zwei Tri-fluormethylgruppen substituiert ist; (n) Perhalo($C_1$-$C_7$)alkyl bedeutet; (o) $C_1$-$C_6$Alkoxy bedeutet; (p) Diphenylmethoxy bedeutet; (q) Cyclo($C_3$-$C_6$)alkoxy bedeutet, welches gegebenenfalls mit einem oder zwei Alkylen substituiert ist; (r) Phenoxy bedeutet, gegebenenfalls substituiert mit einem, zwei oder drei Halogenen oder einer oder zwei Trifluormethylgruppen; (s) Benzyloxy bedeutet, welches gegebenenfalls mit einem, zwei oder drei Halogenen oder $C_1$-$C_4$Alkoxygruppen oder einer oder zwei Trifluormethylgruppen substituiert ist; (t) heteroaromatische Gruppen, welche gegebenenfalls mit einem, zwei oder drei Halogenen oder einer oder zwei Trifluormethylgruppen substituiert ist; (u) Phenyl bedeutet, gegebenenfalls mit einem, zwei oder drei Halogenen oder einer oder zwei Trifluormethylgruppen, Amino, Alkyl($C_1$-$C_3$)amino, Dialkyl($C_1$-$C_3$)amino, $C_1$-$C_5$Alkyl oder $C_1$-$C_3$Alkoxy substituiert ist; (v) N-Morpholinyl($C_1$-$C_4$)-alkyl bedeutet; (w) N-Piperidinyl($C_1$-$C_4$)alkyl bedeutet; (x) N-Pyrro-lidinyl($C_1$-$C_4$)alkyl bedeutet; (y) überbrückte polycyclische Kohlenwasserstoff-Substituenten von sechs bis zehn Kohlenstoffen bedeutet, welche gegebenenfalls mit einer, zwei oder drei ($C_1$-$C_3$)Alkylgruppen substituiert sind; oder (z) Naphthyl($C_1$-$C_3$)alkyl bedeutet, welches gegebenenfalls mit einem, zwei oder drei $C_1$-$C_4$Alkyl, $C_1$-$C_4$Alkoxy, Halogenen oder Trifluormethyl substituiert ist;

worin Y ein Sauerstoffatom, ein Schwefelatom oder eine N-Z-Gruppe bedeutet;

worin Z Wasserstoff, $C_1$-$C_4$Alkyl; Acetyl, Benzoyl, Phenyl, gegebenenfalls substituiert mit einem, zwei oder drei $C_1$-$C_4$Alkyl, $C_1$-$C_3$Alkoxy, Halogen, Trifluormethyl, $C_2$-$C_6$Di-alkylamino, $C_1$-$C_3$Alkylthio, Nitro oder Phenoxy, welches gegebenenfalls mit einem, zwei oder drei $C_1$-$C_4$Alkyl, $C_1$-$C_3$Alkoxy, Ha-logen oder Trifluormethyl substituiert ist; oder Phenyl($C_1$-$C_4$)alkyl, gegebenenfalls substituiert mit einem, zwei oder drei $C_1$-$C_4$Alkyl, $C_1$-$C_4$Alkoxy, Halogen oder Trifluormethyl bedeutet;

worin $R_1$ Wasserstoff, Hydroxy, $C_1$-$C_4$Alkyl, $C_1$-$C_3$Alkoxy, $C_1$-$C_3$Alkylthio, Halogen, Nitro, Benzyloxy oder Trifluormethyl bedeutet;

worin $R_2$ Wasserstoff, Hydroxy, $C_1$-$C_4$Alkyl, $C_1$-$C_3$Alkoxy, $C_1$-$C_3$-Alkylthio, Halogen oder Trifluormethyl bedeutet,

worin $R_3$ Wasserstoff, $C_1$-$C_4$Alkyl, Cyclo($C_3$-$C_6$)alkyl, gegebenenfalls substituiert mit einem, zwei oder drei $C_1$-$C_3$Alkyl, Phenyl, gegebenenfalls substituiert mit einem, zwei oder drei $C_1$-$C_4$Alkyl, Halogen, Trifluormethyl oder $C_1$-$C_3$Alkoxy, Phenyl($C_1$-$C_3$)alkyl, gegebenenfalls substituiert mit einem, zwei oder drei $C_1$-$C_4$Alkyl, Halogen, Trifluormethyl oder $C_1$-$C_3$Alkoxy oder 1,3-Dioxacyclohexan-5-yl bedeutet, und n 0 oder 1 bedeutet,

mit den Provisos, daß

(i) n ist 0, wenn Y ein Schwefelatom oder eine N-Z-Gruppe bedeutet;

(ii) wenn X N-Propyl bedeutet, $R_3$ Methyl, $R_1$ und $R_2$ Wasserstoff bedeuten, die Verbindung etwas Anderes als ein Indol-3-yl-acylhydrazon ist;

(iii) wenn X Cyclopropyl, $R_2$ 2-Methyl, $R_1$ und $R_3$ Wasserstoff bedeuten, die Verbindung etwas Anderes als ein Indol-3-yl-acylhydrazon bedeutet;

(iv) wenn X Cyclohexylmethyl, $R_1$ und $R_2$ Wasserstoff, $R_3$ Ethyl bedeuten, bedeutet die Verbindung etwas Anderes als 1-Benzylindol-3-yl-acylhydrazon;

(v) wenn X t-Butoxy bedeutet, ist die Verbindung ein 2-Benzofuranylacylhydrazon, und $R_1$ und $R_2$ sind Wasserstoff und $R_3$ etwas Anderes als Methyl;
(vi) wenn X Ethoxy bedeutet, $R_1$, $R_2$ und $R_3$ Wasserstoff sind, ist die Verbindung etwas Anderes als 1-Acetylindol-3-yl-acylhydrazon;
(vii) wenn X Ethoxy ist, $R_1$ und $R_2$ Wasserstoff sind, $R_3$ Ethyl ist, ist die Verbindung etwas Anderes als 1-Benzylindol-3-yl-acylhydrazon;
(viii) wenn X Ethoxy ist, $R_1$ und $R_2$ Wasserstoff sind, $R_3$ Methyl ist, ist die Verbindung etwas Anderes als ein 3-Benzothiofuran-acylhydrazon;
(ix) wenn X Phenoxy ist, $R_1$ und $R_2$ Wasserstoff sind und $R_3$ Methyl ist, ist die Verbindung etwas Anderes als ein 2-Benzofuranyl-acylhydrazon;
(x) wenn X Benzyloxy ist und $R_1$, $R_2$ und $R_3$ Wasserstoff sind, ist die Verbindung etwas Anderes als ein Indol-3-yl-acylhydrazon;
(xi) wenn X 4-Ethoxyphenyl oder 4-Chlorphenyl ist, $R_1$ und $R_2$ Wasserstoff sind und $R_3$ Methyl ist, ist die Verbindung etwas Anderes als ein 3-Benzothienyl-acylhydrazon;
(xii) wenn X 4-Chlorphenyl ist, $R_1$ und $R_2$ Wasserstoff sind, $R_3$ Ethyl ist, ist die Verbindung etwas Anderes als 1-Benzylindol3-yl-acylhydrazon;
(xiii) wenn X 4-Methylphenyl, 3,4-Dimethoxyphenyl oder 3-Methoxyphenyl ist und $R_1$, $R_2$ und $R_3$ Wasserstoff sind, ist die Verbindung etwas Anderes als ein Indol-3-yl-acylhydrazon;
(xiv) wenn X 4-Ethoxyphenyl ist, $R_1$, $R_2$ und $R_3$ Wasserstoff sind, ist die Verbindung etwas Anderes als 1-Acetylindol-3-yl-acylhydrazon; und
(xv) wenn $R_1$ Benzyloxy ist, ist X etwas Anderes als Alkyl; und
(xvi) die Acylhydrazone oder Hydrate der pharmazeutisch verträglichen Salze davon etwas Anderes als
Essigsäure-[(1H-indol-3-yl)methylen]hydrazid, (CA-RN-1016-49-5);
4-Aminobenzoesäure-[(1H-indol-3-yl)methylen]hydrazid, (CA-RN 10245-42-8);
4-Methoxybenzoesäure-[(1-methylindol-2-yl)methylen]hydrazid, (CA RN 15565-86-3);
4-Morpholinessigsäure-(2-benzofuranylmethylen)hydrazid, (CA-RN 72627-58-8);
(2-Chlorphenoxy)essigsäure (1H-indol-3-yl-methylen)hydrazid, (CA-RN 81111-25-3);
(4-Chlorphenoxy)essigsäure-(1H-indol-3-yl-methylen)hydrazid, (CA-RN 81111-26-4);
(2-Chlorphenoxy)essigsäure-[(2-methoxy-1H-indol-3-yl)methylen]hydrazid, (CA-RN 81122-74-9);
(4-Chlorphenoxy)essigsäure-[(2-methoxy-1H-indol-3-yl)methylen]hydrazid (CA-RN 8122-75-0);
Methyl-(1H-indol-3-yl-methylen)carbazat (CA-RN 88692-99-3);
Phenylmethyl-[1-(1H-indol-3-yl)propyliden]carbazat (CA-RN 32947-16-3);
1-Piperidinessigsäure-[(2-methyl-3-benzofuranyl)methylen]hydrazid (CA RN 35806-44-1);
1-Pyrrolidinessigsäure-(2-benzofuranylmethylen)hydrazid (CA RN 35806-70-3);
1-Piperidinessigsäure-(2-benzofuranylmethylen)hydrazid (CA RN 35806-85-0);
1-Pyrrolidinessigsäure-[(2-methyl-3-benzofuranyl)methylen]hydrazid (CA RN 35802-00-9);
Ameisensäure-[(1H-indol-2-yl)-methylen]hydrazid (CA RN 50335-88-1);
Ethyl-(1H-indol-2-yl-methylen)carbazat (CA RN 50335-89-2); Ethyl-[1-(2-benzofuranyl)ethyliden]-carbazat (CA RN 56968-94-6);
Ethyl-[1-(5-chlor-2-benzofuranyl)ethyliden]carbazat (CA RN 56968-95-7);
1,1-Dimethylethyl-(1H-indol-3-yl-methylen)carbazat (CA RN 57699-52-2);
4-Aminobenzoesäure-[(1H-indol-3-yl)methylen]hydrazid (CA RN 15641-21-1);
Buttersäure-[(1H-indol-3-yl)methylen]hydrazid (CA RN 15641-24-4);
Propionsäure-[(1H-indol-3-yl)methylen]hydrazid (CA RN 15641-25-5);
Ethyl-[(1H-indol-3-yl)methylen]carbazat (CA RN 15641-27-7);
Ameisensäure-[(1-methylindol-2-yl)methylen]hydrazid (CA RN 15641-28-8);
Essigsäure-[(1-methylindol-2-yl)methylen]hydrazid (CA RN 15641-29-9);
Propansäure-[(1-methylindol-2-yl)methylen]hydrazid (CA RN 15641-30-2);
Butansäure-[(1-methylindol-2-yl)methylen]hydrazid (CA RN 15641-31-3);
2-Methylpropansäure-[(1-methylindol-2-yl)methylen]hydrazid (CA RN 15641-32-4);
Phenylessigsäure-[(1-methylindol-2-yl)methylen]hydrazid (CA RN 15641-34-6);
4-Chlorbenzoesäure-[(1-methylindol-2-yl)methylen]hydrazid (CA RN 15641-36-8);
1-Naphthalinessigsäure-[(1-methylindol-2-yl)methylen]hydrazid (CA RN 15641-38-0);
Ethyl-[(1-methylindol-2-yl)methylen]carbazat (CA RN 15565-88-5);
4-Chlorbenzoesäure-[(1H-indol-3-yl)methylen]hydrazid (CA RN 15641-01-7);
2-Chlorbenzoesäure-[5(1H-indol-3-yl)methylen]hydrazid (CA RN 15641-03-9);
3-Chlorbenzoesäure-[(1H-indol-3-yl)methylen]hydrazid (CA RN 15641-04-0);

2,4-Dichlorbenzoesäure-[(1H-indol-3-yl)methylen]hydrazid (CA RN 15641-05-1);
4-Brombenzoesäure-[(1H-indol-3-yl)methylen]hydrazid (CA RN 15641-06-2);
2-Methylpropansäure-[(1H-indol-3-yl)methylen]hydrazid (CA RN 15641-12-0);
4-Methoxybenzoesäure-[(1H-indol-3-yl)methylen]hydrazid (CA RN 15641-15-3);
Phenylessigsäure-[(1H-indol-3-yl)methylen]hydrazid (CA RN 15641-17-5);
1-Naphthalinessigsäure-[(1H-indol-yl)methylen]hydrazid (CA RN 15641-18-6);
2-Furonsäure-[(1H-indol-3-yl)methylen]hydazid (CA RN 15641-19-7);
2-Naphthalinessigsäure-[(1H-indol-3-yl)methylen]hydrazid (CA RN 15718-86-2);
Ethyl-[1-(1H-indol-3-yl)ethyliden]carbazat (CA RN 32927-03-0);
Phenylmethyl-3-[(1H-indol-3-yl)methylen]carbazat (CA RN 32927-05-2);
Phenylmethyl-[1-(1H-indol-3-yl)ethyliden]carbazat (CA RN 32927-06-3); oder
Ethyl-[(1-methylindol-3-yl)methylen]carbazat (CA RN 32927-89-2),
sind.

**2.** Verfahren nach Anspruch 1, worin n 0 ist.

**3.** Verfahren nach Anspruch 2, worin Y NH bedeutet.

**4.** Verfahren nach Anspruch 3, worin das Acylhydrazon ein 1H-Indol-3-yl-acylhydrazon ist.

**5.** Verfahren nach einem der vorhergehenden Ansprüche, worin X Cyclobutyl, t-Butoxy, Ethoxy, Ethyl oder 3-Pyridinyl ist.

**6.** Verfahren nach einem der vorhergehenden Ansprüche, worin $R_1$ und $R_2$ unabhängig voneinander aus Wasserstoff, Methyl und Brom gewählt sind.

**7.** Verfahren nach einem der vorhergehenden Ansprüche, worin $R_3$ Wasserstoff bedeutet.

**8.** Verfahren nach Anspruch 1, worin das Acylhydrazon 1,1-Dimethylethyl-[(1H-indol-3-yl)methylen]-carbazat (Verb.4);
Nicotinsäure-[(1H-indol-3-yl)methylen]hydrazid (Verb.13);
Ethyl-[(5-brom-1H-indol-3-yl)methylen]carbazat (Verb.52);
Cyclobutancarbonsäure-[(1H-indol-3-yl)methylen]hydrazid (Verb.66);
Ethyl-[(2-methyl-1H-indol-3-yl)methylen]carbazat (Verb.67);
Butansäure-[(2-methyl-1H-indol-3-yl)methylen]hydrazid (Verb.69);
Propansäure-[(2-methyl-1H-indol-3-yl)methylen]hydrazid (Verb.70);
Nicotinsäure-[(2-methyl-1H-indol-3-yl)methylen]hydrazid (Verb.96);
1,1-Dimethylethyl-[(2-methyl-1H-indol-3-yl)methylen]hydrazid (Verb.97);
Propansäure-[(1-acetylindol-3-yl)methylen]hydrazid (Verb.145); oder
Phenylessigsäure-[(2-methyl-1H-indol-3-yl)methylen]hydrazid (Verb.167),
ist.

**9.** Verfahren nach Anspruch 1, worin das Acylhydrazon Essigsäure-[(1-phenylmethylindol-3-yl)propyliden]-hydrazid (Verb.64) ist.

**10.** Verfahren nach Anspruch 1, worin das Acylhydrazon Ameisensäure-[(1-(3-benzofuranyl)ethyliden]-hydrazid (Verb.81) ist.

**11.** Verfahren nach Anspruch 1, worin das Acylhydrazon Buttersäure-[1-(3-benzothienyl)ethyliden]hydrazid (Verb.118) ist.

**12.** Verfahren nach Anspruch 1, worin das Acylhydrazon Ethyl[1-(2-oxo-2H-1-benzopyran-3-yl)ethyliden]-carbazat (Verb.248); oder Buttersäure-[1-(2-oxo-2H-1-benzopyran-3-yl)-ethyliden]hydrazid (Verb.250) ist.